# EUROPEAN PATENT SPECIFICATION

(11) **EP 1 692 519 B1**
(45) Date of publication and mention of the grant of the patent: **02.06.2010**
(21) Application number: 04806548.6
(22) Date of filing: 03.12.2004
(51) Int. Cl.: G01N 33/68

(54) **METHOD FOR DIAGNOSING DISEASES BASED ON LEVELS OF ANTI-GLYCAN ANTIBODIES**
DIAGNOSTIKVERFAHREN MITTELS MESSUNGEN DER ANTIGLYKAN-ANTIKÖRPER-SPIEGELN
METHODE DE DIAGNOSTIC DE MALADIES SUR LA BASE DES NIVEAUX D'ANTICORPS CONTRE DES GLYCANES

(30) Priority: 03.12.2003 US 728227; 11.05.2004 US 843033
(43) Date of publication of application: 23.08.2006
(73) Proprietor: Glycominds Ltd., Lod 71291 (IL)
(72) Inventor: DOTAN, Nir, 76452 Rehovot (IL); DUKLER, Avinoam, Moddi'in (IL)
(74) Representative: Peter, Beate
(86) International application number: PCT/IB2004/004389
(87) International publication number: WO 2005/054853

(56) References cited:
- WO-A-92/16837
- WO-A-02/064556
- WO-A-2004/015420
- US-A- 5 932 429
- US-A1- 2003 143 649
- US-B1- 6 218 129
- SENDID B ET AL: "SPECIFIC ANTIBODY RESPONSE TO OLIGOMANNOSIDIC EPITOPES IN CROHN'S DISEASE" CLINICAL AND DIAGNOSTIC LABORATORY IMMUNOLOGY, AMERICAN SOCIETY FOR MICROBIOLOGY, US, vol. 3, no. 2, 1996, pages 219-226, XP000910700 ISSN: 1071-412X
- SCHWARZ MIKAEL ET AL: "A new kind of carbohydrate array, its use for profiling antiglycan antibodies, and the discovery of a novel human cellulose-binding antibody." GLYCOBIOLOGY, vol. 13, no. 11, November 2003 (2003-11), pages 749-754, XP002335360 ISSN: 0959-6658
- WANG DENONG: "Carbohydrate microarrays." PROTEOMICS, vol. 3, no. 11, November 2003 (2003-11), pages 2167-2175, XP002335361 ISSN: 1615-9853

## Description

### FIELD OF THE INVENTION

The invention relates generally to a method for diagnosing diseases by detecting levels of antibodies to glycans in a subject. More particularly, the invention relates to methods useful in diagnosing digestive diseases such as Crohn's disease (CD).

### BACKGROUND OF THE INVENTION

Inflammatory bowel disease (IBD), which occurs world-wide and afflicts millions of people, is the collective term used to describe two gastrointestinal disorders of unknown etiology: Crohn's disease (CD) and ulcerative colitis (UC). IBD and irritable bowel syndrome (IBS) will affect one-half of all Americans during their lifetime, at a cost of several billion dollars. A primary determinant of these high medical costs is the difficulty in diagnosing digestive diseases. The cost associated with IBD and IBS is compounded by lost productivity, with persons suffering front these disorders missing an average of at least eight more days of work annually than persons not suffering from these disorders.

Symptoms associated with CD include, e.g., abdominal pain, chronic diarrhea, rectal bleeding, weight loss, and cramping. These symptoms are also found in irritable bowel syndrome and other inflammatory bowel diseases. This makes definitive diagnosis of CD extremely difficult. In fact, only about one-tenth of the several million people suspected of suffering from CD are actually diagnosed with the disease The difficulty in differentially diagnosing CD from other digestive diseases like UC and IBS hampers early and effective treatment of these diseases.

CD (ileitis regionalis or ileitis terminalis) may affect any part of the gut with the ileum and colon as the most commonly affected sites. In CD, the inflammation is asymmetrical and segmental, with areas of both healthy and diseased tissue. In contrast, UC (hemorrhagic idiopathic proctocolitis) is characterized by symmetrical inflammation - restricted to mucosa and submucosa - ascending uninterrupted from rectum to colon.

CD is typically diagnosed using upper or lower GI endoscopy and/or by X-ray examination of the small intestine including the ileum. In CD, no typical endoscopic picture is observed while in UC, the typical pattern detected is an inflamed red mucosa with bleeding. CD biopsy specimens further reveal transmural inflammation with lymphocytes, macrophages and plasma cells while mucosal/submucosal inflammation with granulocytes, eosinophiles and plasma cells is typical of UC. When inflammation is limited to the colon, it can be very difficult to differentiate between UC or colon-restricted UC (which also known as CD colitis).

Antiphospholipid syndrome (APS) is characterized by venous or arterial thrombosis, recurrent miscarriages, and thrombocytopenia, which is a low number of blood platelets that can lead to bleeding, seen as bruising and tiny red dots on the skin. Patients with APS also may experience symptoms of stroke such as transient ischemic attacks (TIAs).

Antiphospholipid syndrome is typically diagnosed based on these clinical manifestations and on laboratory test results. A blood sample is analyzed for the presence of antibodies that react with naturally occurring proteins complexed with phospholipids. These are called antiphospholipid antibodies or anticardiolipin antibodies (cardiolipin is one type of phospholipid used in lab tests). Sometimes these antibodies are called lupus anticoagulants when clotting assays are used for their detection.

### SUMMARY OF THE INVENTION

The invention is based in part on the discovery that patients with Crohn's disease (CD) or anti-phospholipid syndrome (APS) have elevated serum levels of certain IgG, IgA, and IgM isotype antibodies specific for certain glycan structures, as compared to such levels in healthy individuals or in individuals with other types of gastrointestinal diseases.

In particular the present invention provides a method useful in diagnosing Crohn's disease in a subject, the method comprising
identifying in a test sample from a subject with a symptom of Crohn's disease a specific anti-glycan antibody, wherein said specific anti-glycan antibody is an anti-GlcNAc(β,1-4)GlcNAc(β) antibody or an anti-Glc(β,1-3)Glc(β) antibody,
wherein the identification of elevated levels of said antibody in said test sample relative to a control sample indicates the subject is likely to have Crohn's disease.

Among the advantages of the disclosure is a highly sensitive serological testing method for definitively distinguishing CD from other digestive diseases, even when inflammation is limited to the colon. The highly sensitive primary screening assays of the disclosure provide physicians with an inexpensive way to rapidly distinguish individuals with CD from non-diseased individuals or individuals having UC or IBS. This facilitates earlier and more appropriate therapeutic intervention and minimizes uncertainty for patients and their families. In one aspect, the disclosure provides a method of diagnosing CD in a subject by providing a test sample from the subject and detecting in the test sample at least one of the following anti-glycan antibodies: an anti-Glc(β) antibody, an anti-Glc(β,1-4)Glc(β) antibody, an anti-Glc(β,1-3)Glc(β) antibody, an anti-GlcNAc(β) 6-sulfate antibody, an anti-Dextran antibody, an anti-Xylan antibody, an anti-GlcNAc(β,1-4)GlcNAc(β) antibody, an anti-Gal 3-sulphate (β) antibody, an anti-GlcNAc(β,1-3)GalNAc(β) antibody, an anti-GlcNAc(β,1-3)Gal(β,1-4)Glc(β) antibody, an anti-Gal(α) antibody, an anti-Gal(β) antibody, an anti- GalNAc(α) antibody, an anti-Glc(α) antibody, an anti-Gal(β,1-6)Gal(β) antibody, or an anti-GlcNAc(β,1-6)GalNAc(α) antibody. The presence of one or more of the antibodies in the test sample identifies the subject as having CD.

In some embodiments, levels of the anti-glycan antibody or antibodies in the test sample are compared to the levels of anti-glycan antibodies in a control sample. The control sample is chosen from a group that includes one or more individuals known to have or not to have a gastrointestinal disorder, or to have or not to have a gastrointestinal disorder other than CD. When the control sample is from an individual or individuals that do not have CD or has a gastrointestinal disease other than CD, elevated levels in the test sample relative to the control sample identifies the subject as having CD.

In some embodiments, the control sample is from one or more individuals with a gastrointestinal disorder that is irritable bowel syndrome, ulcerative colitis or other digestive diseases. In some embodiments, the control sample is from one or more individuals that do not have a gastrointestinal disorder.

In various embodiments, at least 2, 3, 4, 5, 6, 7, 8, 9, 10, 11, 12, 13, 14, or all of these antibodies are detected.

In some embodiments, the method further includes determining whether the test sample has an anti-Mannan antibody, which is also known as an anti-Saccharomyces cerevisiae antibody (ASCA). The presence of the anti-Mannan antibody in the sample identifies the subject as having CD.

In some embodiments, the method further includes determining whether the test sample has an anti-neutrophil cytoplasmic antibodies (ANCA). The presence of ANCA indicates that the subject does not have CD but may have Ulcerative Colitis.

The test sample may be, e.g., a biological fluid. Examples of biological fluids include, e.g., whole blood, serum, plasma, spinal cord fluid, urine, or saliva.

In some embodiments, one, two, three, four or all five of an anti-Glc(β,1-3)Glc(β) antibody, anti-Man(α,1-3)Man(α) antibody, anti-Man(α,1-3) [Man(α,1-6)]Man(a) antibodies, anti-Man(α) and/or anti-Mannan antibodies are detected.

The method may optionally include determining the isotype of the antibody. For example the method may include determining whether the antibody is an IgM, IgA, or IgG-type antibody. In some embodiments, the method is used to identify and compare one or more of an anti-Glc(β)IgG antibody, an anti-Glc(β,1-3)Glc(β) IgG antibody, an anti-Glc(β,1-4)Glc(β) IgG antibody, an anti-GlcNAc(β) 6-sulfate IgG antibody, an anti-Man(α) IgG antibody, an anti-Man(α,1-3) [Man (α,1-6)] Man(β) IgG antibody, an anti- Man(α,1-3)Man(α) IgG antibody, an anti-Mannan IgG antibody, an anti-Mannan IgA antibody, an anti-Xylan IgG antibody, or an anti-Man(α,1-2)Man(α) IgG antibody.

In some embodiments, a subject is scored as having CD if the test sample has elevated levels of IgG anti-Glc(β,1-3)Glc(β), IgG anti-Man(α,1-3) Man(α), IgG anti Mannan (ASCA) antibodies, or IgA anti Mannan (ASCA) antibodies, but does not have elevated levels of ANCA relative to levels in a control sample.

In some embodiments, a subject is scored as having IBD if the test sample has elevated levels of IgG anti-Glc(β,1-3)Glc(β), IgG anti anti-Man(α,1-3)Man(α), IgG anti Mannan (ASCA) antibodies, IgA anti Mannan (ASCA) antibodies, or ANCA relative to levels in a control sample taken from a subject known to not have CD.

In some embodiments, a subject is scored as having a non-IBD digestive disease if the test sample has elevated levels of IgA anti-GlcNAc(β,1-4)GlcNAc(β), and low levels of anti Mannan (ASCA) antibodies relative to a control sample.

In some embodiments, the anti-glycan antibody or antibodies are detected using a fluorescent antibody, or are detected using an enzyme-linked immunoabsorbent assay (ELISA).

The test sample may be, e.g., a biological fluid. Examples of biological fluids include, e.g., whole blood, serum, plasma, spinal cord fluid, urine, or saliva.

The method may optionally include determining the isotype of the antibody. For example the method can include determining whether the antibody is an IgM, IgA, or IgG-type antibody.

In another aspect, the invention provides a method useful in diagnosing CD in a subject. The method includes providing a test sample from a subject and determining whether an anti-glycan antibody is present in the test sample. At least one anti-glycan antibody is an IgG Glc(β,1-3)Glc(β) antibody or an IgG anti GlcNAc(β,1-4)GlcNAc(β) antibody. The presence of at least one antibody in the test sample identifies the subject as having CD.

In some embodiments, levels of the anti-glycan antibody or antibodies in the test sample are compared to the levels of anti-glycan antibodies in a control sample. The control sample is chosen from a group that includes one or more individuals known to have or not to have a gastrointestinal disorder, or to have or not to have a gastrointestinal disorder other than CD. When the control sample is from an individual or individuals that do not have CD, or has a gastrointestinal disease other than CD, elevated levels in the test sample relative to the control sample identifies the subject has CD.

In some embodiments, the control sample is from one or more individuals with a gastrointestinal disorder that is irritable bowel syndrome or ulcerative colitis or other digestive diseases. In some embodiments, the control sample is from one or more individuals that do not have a gastrointestinal disorder.

In a further aspect, the disclosure provides a method of differentially diagnosing CD or IBD in a subject. The method includes providing a test sample from a subject and determining whether the sample has an antibody that is an anti-neutrophil cytoplasmic antibody (ANCA), an IgG anti-Glc(β,1-3)Glc(β)antibody, an IgG ASCA and/or IgA ASCA. The absence of ANCA and the presence of at least one of the IgG anti-Glc(β,1-3)Glc(β) IgG ASCA, and IgA ASCA antibodies in the test sample indicates the subject has CD, and the presence of at least one of the antibodies in the test sample indicates the subject has IBD.

In some embodiments, the anti-glycan antibody or antibodies are detected using a fluorescent antibody, or are detected using an enzyme-linked immunoabsorbent assay (ELISA).

The test sample can be, e.g., a biological fluid. Examples of biological fluids include, e.g., whole blood, serum, plasma, spinal cord fluid, urine, or saliva.

The disclosure additionally provides a method useful in differentially diagnosing CD colitis and ulcerative colitis in a subject. The method involves determining levels of at least one an anti-glycan antibody in a sample from a subject. The anti-glycan antibody may be one or more of an IgG anti-Gal(α,1-4)GlcNAc(α) antibody, an IgG anti-Gal(β,1-4)GlcNAc(β) antibody, an IgG anti- GalNAc(α) antibody, an IgG anti-Glc(α) antibody, an IgG anti-Glc(β) antibody, an IgG anti-GlcNAc(β,6-Sulphate) antibody, an IgG anti-GlcNAc(β) antibody, an IgG anti-GlcNAc(β,1-6)GalNAc(α) antibody, an IgA anti-Gal(α,1-3)Gal(β,1-4)GlcNAc(β,1-3)Gal(β,1-4)Glc(β)antibody, an IgA anti-Gal(α,1-4)Gal(β,1-4), Glc(β) antibody, an IgA anti-Gal(β) antibody, an IgA anti-Gal(β,1-3)[GlcNAc(β,1-6)]GalNAc(α) antibody, an IgA anti-Gal(β,1-3)GlcNAc(β) antibody, an IgA anti-Gal(β,1-6)Gal(β) antibody, an IgA anti-GalNAc(α) antibody, an IgA anti-GalNAc(β) antibody, IgA an anti-Glc(β) antibody, an IgA anti-Glc(β,1-3)Glc(β) antibody, an IgA anti-GlcNAc(β) antibody, an IgA anti-GlcNAc(β,1-3)Gal(β, 1-4)Glc(β) antibody, an IgA anti-GlcNAc(β,1-3)GalNAc(α) antibody, an IgA anti-GlcNAc(β,1-4)GlcNAc(β) antibody, an IgA anti-GlcNAc(β,1-6)GalNAc(α) antibody, or an IgA anti-Xyl(β) antibody. The presence of at least one antibody in the test sample indicates the subject has CD colitis.

In some embodiments, the method further includes comparing the levels of the at least one anti-glycan antibody in the test sample to the levels of the at least one anti-glycan antibody in a control sample, such that the control sample is one or more individuals known to have or not to have Crohn's disease colitis or one or more individuals known to have or not to have ulcerative colitis (UC).

In some embodiments, the method involves determining whether an additional anti-glycan antibody or antibodies are present in the sample. The additional anti-glycan antibody is one or more of an IgG anti-Gal(α) antibody, an IgG anti-Man(α) antibody, an IgG anti-Man(α,1-3)Man(α,1-6)Man(β) antibody, an IgG anti-Man(α,1-3)Man(α,1-6)Man(β) antibody, an IgG anti-Man(α,1-3)Man(α) antibody, an IgA anti-Man(α) antibody, an IgA anti-Man(α,1-2)Man(α) antibody, an IgA anti-Man(α,1-3)Man(α,1-6)Man(β) antibody, an IgA anti-Man(β,1-3)Man(α) antibody, an IgA anti-Man(α,1-6)Man(α) antibody, an IgA anti-Man(β) antibody, or an IgA anti-X(α) antibody. The presence of the additional antibody or antibodies in the test sample identifies the subject as having CD colitis.

In some embodiments, the additional antibody or antibodies is an IgA anti-GlcNAc(β,1-4)GlcNAc(β) antibody and/or and IgG anti-Man(α,1-3)Man(α) antibody.

In some embodiments, the method includes detecting at least two, three, four, five, six seven, eight, nine, ten, eleven or twelve of the antibodies.

In some embodiments, the test sample is a biological fluid (e.g., whole blood, serum, plasma, urine, or saliva).

Also provided is a method for differentially diagnosing inflammatory bowel disease (IBD) or non-IBD digestive disease (NIC) in a subject. The method involves providing a test sample from a subject with symptoms of NIC or IBD and determining if anti-chitobioside (GlcNAc(β,1-4)GlcNAc(β)) carbohydrate antibodies (ACCA) and anti-mannan (ASCA) antibodies are present in the sample. The presence of ACCA antibodies and the absence of ASCA antibodies in the sample identifies the subject as having NIC.

In some embodiments, the levels of anti chitobioside (GlcNAc(β,1-4)GlcNAc(β)) carbohydrate antibodies (ACCA) and/or anti-mannan (ASCA) antibodies are determined by comparing levels of the antibodies to such levels in a reference sample from a subject known to have IBD. A higher level of ACCA antibodies and a lower level of ASCA antibodies in the test sample relative to the reference sample identifies the patient as having NIC.

In some embodiments, the method further includes determining whether the test sample has anti-laminarobioside (Glc(β,1-3)Glc(β)) Carbohydrate Antibodies (ALCA) antibodies, such that the absence of ALCA antibodies in the sample identifies the subject as having NIC.

In some embodiments, the anti-glycan antibody or antibodies are detected using a fluorescent antibody or an enzyme-linked immunoabsorbent assay (ELISA).

The test sample may be, e.g., a biological fluid. Examples of biological fluids include, e.g., whole blood, serum, plasma, spinal cord fluid, urine, and saliva.

The disclosure additionally provides reagents for detecting anti-glycan antibodies that reveal the presence of CD. The reagents include one or more carbohydrates that specifically react with an anti-Glc(β) antibody, an anti-Glc(β,1-4)Glc(β) antibody, an anti-Glc(β,1-3)Glc(β) antibody, an anti-GlcNAc(β) 6-sulfate antibody, an anti-Man(α,1-2)Man(α) antibody, an anti-Man(α,1-3)Man(α) antibody, an anti-Man(α,1-6)Man(α) antibody, an anti-Man(α) antibody, an anti-Man(α,1-3)[Man(α,1-6)]Man(α), an anti-Mannan antibody, an anti-Dextran antibody, an anti-Xylan antibody, an anti-GlcNAc(β, 1-4)GlcNAc(β) antibody, an anti-Gal 3-sulphate(β) antibody, an anti-aGlcNAc(β,1-3)GalNAc(β)antibody, an anti-GlcNAc(β,1-3)Gal(β,1-4)Glc(β) antibody, and/or an anti-Gal(α,1-3)Gal(β,1-4)GlcNAc(β) antibody. In some embodiments, the reagents are attached to a solid phase.

Also within the disclosure are arrays that include reagents (preferably carbohydrate reagents) that specifically detect the disease-detecting antibodies disclosed herein. For example, an array useful for detecting CD can include one or more reagents that detect an anti-Glc(β) antibody, an anti-Glc(β,1-4)Glc(β) antibody, an anti-Glc(β,1-3)Glc(β) antibody, an anti-GlcNAc(β) 6-sulfate antibody, an anti-Man(α,1-2)Man(α) antibody, an anti-Man(α,1-3)Man(α) antibody, an anti-Man(α,1-6)Man(α) antibody, an anti-Man(α) antibody, an anti-Man(α,1-3)[Man(α,1-6)]Man(α), an anti-Mannan antibody, an anti-Dextran antibody, an anti-Xylan antibody, an anti-GlcNAc(β,1-4)GlcNAc(β) antibody, an anti-Gal 3-sulphate(β) antibody, an anti-GlcNAc(β,1-3)GalNAc(β) antibody, an anti-GlcNAc(β,1-3)Gal(β,1-4)Glc(β) antibody, or an anti-Gal(α,1-3)Gal (β,1-4)GlcNAc(β) antibody.

In some embodiments, the reagents that are used to specifically bind and detect those anti glycans antibodies are the specific glycan structures. In other embodiments, the reagents are other molecules or macromolecules that include the specific glycan structure. For example, the anti-Glc(β,1-3)Glc(β) antibody can be detected using the polysaccharide β-D(1-3) Glucan, a polymer of glucose units connected in a (β,1-3) glycosidic bond. Thus, the glycan itself may be used for detecting the corresponding antibody or antibodies, as may any carbohydrate, peptide, protein, or any other molecular structure that includes the glycan.

In some embodiments, the reagents that are used to specifically bind and detect the anti glycans antibodies of the invention are peptides that mimic the carbohydrate antigens of the invention. The peptides can be used to identify specific anti-glycan antibodies.

The array may additionally include a reagent or reagent, e.g., a carbohydrate reagent or reagents, that detect an anti-Mannan (ASCA) antibody or an ANCA.

In some embodiments, the glycans are attached to the array via a linker. A suitable linker includes at least one ethylene glycol derivative, at least two cyanuric chloride derivatives and an anilino group.

In some embodiment, at least two of the reagent or reagents are provided at the same location on the addressable array.

In some embodiments, the array includes a reagent, e.g., a glycan reagent that detects an anti-Glc(β,1-3)Glc(β) antibody and/or an IgG anti-Man(α,1-3)Man(α) antibody.

Other arrays provided herein include arrays useful for differentially diagnosing CD or IBD in a subject. The array includes one or more reagents (e.g., glycan or peptide reagents) that detect an anti-neutrophil cytoplasmic antibody (ANCA), an anti-Glc(β,1-3)Glc(β)antibody, an ASCA; or an ASCA. In some embodiments, the array includes, one, two, or three of these reagents.

The disclosure additionally provides an array of reagents (e.g., glycan or peptide reagents) useful for differentially diagnosing CD colitis and ulcerative colitis in a subject. The arrays include one or more reagents that detect an anti-Gal(α,1-4)GlcNAc(α) antibody, an anti-Gal(β,1-4)GlcNAc(β) antibody, an anti-GalNAc(α) antibody, an anti-Glc(α) antibody, an anti-Glc(β) antibody, an anti-GlcNAc(β,6-Sulphate) antibody, an anti-GlcNAc(β) antibody, an anti-GlcNAc(β,1-6)GalNAc(α) antibody, an anti-Gal(α,1-3)Gal(β,1-4)GlcNAc(β,1-3)Gal(β,1-4)Glc(β)antibody, an anti-Gal(α,1-4)Gal(β,1-4) Glc(β) antibody, an anti-Gal(β) antibody, an anti-Gal(β,1-3)[GlcNAc(β,1-6)]GalNAc(α) antibody, an anti-Gal(β,1-3)GlcNAc(β) antibody, an anti-Gal(β,1-6)Gal(β) antibody, an anti-GalNAc(α) antibody, an anti-GalNAc(β) antibody, an anti-Glc(β) antibody, an anti-Glc(β,1-3)Glc(β) antibody, an anti-GlcNAc(β) antibody, an anti-GlcNAc(β,-3)Gal(β,1-4)Glc(β) antibody, an anti-GlcNAc(β,1-3)GalNAc(α) antibody, an anti-GlcNAc(β,1-4)GlcNAc(β) antibody, an anti-GlcNAc(β,1-6)GalNAc(α) antibody, or an anti-Xyl(β) antibody. In some embodiments, the array includes reagents that bind 2, 3, 4, 6, 6, 7, 8, 9, 10,11,12,13,14,15,16,17,18,19,20,21,22,23, or 24 of these antibodies.

The array may additionally include a reagent, e.g, a glycan or peptide reagent, that detects an anti-Gal(α) antibody, an anti-Man(α) antibody, anti-Man(α,1-3)Man(α,1-6)Man(β) antibody, an anti-Man(α,1-3)Man(α,1-6)Man(β) antibody, an anti-Man(α,1-3)Man(α) antibody, an anti-Man(α) antibody, an anti-Man(α,1-2)Man(α) antibody, an anti-Man(α,1-3)Man(α,1-6)Man(β) antibody, an anti-Man(β,1-3)Man(α) antibody, an anti-Man(α,1-6)Man(α) antibody, an anti-Man(β) antibody, and/or an anti- X(α) antibody. In some embodiments, the array includes reagents that bind 2, 3, 4, 6, 6, 7, 8, 9, 10,11, or 12 of these antibodies.

The array may additionally include a reagent (e.g., a glycan or peptide reagent) that detects an anti-GlcNAc(β,1-4)GlcNAc(β) antibody and/or an anti-Man(α,1-3)Man(α) antibody. Also provided by the disclosure is an array useful for differentially diagnosing inflammatory bowel disease (IBD) or non-IBD digestive disease (NIC). The array includes a reagent (e.g., a glycan or peptide reagent) that detects anti chitobioside (GlcNAc(β,1-4)GlcNAc(β)) carbohydrate antibodies (ACCA) and/or anti-mannan (ASCA) antibodies. The array may optionally include a reagent that detects anti-laminarobioside (Glc(β,1-3)Glc(β)) Carbohydrate Antibodies (ALCA).

The disclosure additionally provides kits that include reagents for detecting anti-glycan antibodies that reveal the presence of CD. The kits include one or more carbohydrate reagent(s) that specifically reacts with an anti-Glc(β) antibody, an anti-Glc(β,1-4)Glc(β) antibody, an anti-Glc(β,1-3)Glc(β) antibody, an anti-GlcNAc(β) 6-sulfateantibody, an anti-Man(α,1-2)Man(α) antibody, an anti-Man(α,1-3)Man(α) antibody, an anti-Man(α,1-6)Man(α) antibody, an anti-Man(α) antibody, an anti-Man(α,1-3)[Man(α,1-6)]Man(α), an anti-Mannan antibody, an anti-Dextran antibody, an anti- Xylan antibody, an anti-GlcNAc(β,1-4)GlcNAc(β) antibody, an anti-Gal 3-sulphate(β) antibody, an anti-aGlcNAc(β,1-3)GalNAc(β)antibody, an anti-GlcNAc(β,1-3)Gal(β,1-4)Glc(β) antibody, and/or an anti-Gal(α,1-3)Gal(β,1-4)GlcNAc(β) antibody. The kits may be provided in one or more containers. In some embodiments, the kits contain directions for using the kits to perform the methods described herein. The kits may optionally include reagents for detecting antibody isotypes (e.g., IgA, IgG, and IgM antibodies).

In some embodiments, the kits include reagents that are used to specifically bind and detect those anti glycans antibodies that are the specific glycan structures. In other embodiments, the reagents in the kits are other molecules or macromolecules that include the specific glycan structure. For example, the anti-Glc(β,1-3)Glc(β) antibody can be detected using the polysaccharide β-D(1-3) Glucan, a polymer of glucose units connected in a (β,1-3) glycosidic bond. Thus, the glycan itself may be used for detecting the corresponding antibody or antibodies, as may any carbohydrate, peptide, protein, or any other molecular structure that includes the glycan.

In some embodiments, the kits include reagents that are used to specifically bind and detect ASCA and/or ANCA.

Also provided by the disclosure are kits useful for differentially diagnosing CD or IBD in a subject. The kit includes one or more reagents (e.g., glycan or peptide reagents) that detect an anti-neutrophil cytoplasmic antibody (ANCA), an anti-Glc(β,1-3)Glc(β)antibody, an ASCA, or an ASCA. In some embodiments, the kit includes, one, two, or three of these reagents.

The invention additionally provides a kit of reagents (e.g., glycan or peptide reagents) useful for differentially diagnosing CD colitis and ulcerative colitis in a subject. The kits include one or more reagents that detect an anti-Gal(α,1-4)GlcNAc(α) antibody, an anti-Gal(β,1-4)GlcNAc(β) antibody, an anti-GalNAc(α) antibody, an anti-Glc(α) antibody, an anti-Glc(β) antibody, an anti-GlcNAc(β,6-Sulphate) antibody, an anti-GlcNAc(β) antibody, an anti-GlcNAc(β,1-6)GalNAc(α) antibody, an anti-Gal(α,1-3)Gal(β,1-4)GlcNAc(β,1-3)Gal(β,1-4)Glc(β)antibody, an anti-Gal(α,1-4)Gal(β,1-4) Glc(β) antibody, an anti-Gal(β) antibody, an anti-Gal(β,1-3)[GlcNAc(β,1-6)]GalNAc(α) antibody, an anti-Gal(β,1-3)GlcNAc(β) antibody, an anti-Gal(β,1-6)Gal(β) antibody, an anti-GalNAc(α) antibody, an anti-GalNAc(β) antibody, an anti-Glc(β) antibody, an anti-Glc(β,1-3)Glc(β) antibody, an anti-GlcNAc(β) antibody, an anti-GlcNAc(β,1-3)Gal(β,1-4)Glc(β) antibody, an anti-GlcNAc(β,1-3)GalNAc(α) antibody, an anti-GlcNAc(β,1-4)GlcNAc(β) antibody, an anti-GlcNAc(β,1-6)GalNAc(α) antibody, or an anti-Xyl(β) antibody. In some embodiments, the kit includes reagents that bind 2, 3, 4, 6, 6, 7, 8, 9, 10, 11, 12, 13, 14, 15, 16, 17, 18, 19, 20, 21, 22, 23, or 24 of these antibodies.

The kit may additionally include a reagent, e.g, a glycan or peptide reagent, that detects an anti-Gal(α) antibody, an anti-Man(α) antibody, anti-Man(α,1-3)Man(α,1-6)Man(β) antibody, an anti-Man(α,1-3)Man(α,1-6)Man(β) antibody, an anti-Man(α,1-3)Man(α) antibody, an anti-Man(α) antibody, an anti-Man(α,1-2)Man(α) antibody, an anti-Man(α,1-3)Man(α,1-6)Man(β) antibody, an anti-Man(β,1-3)Man(α) antibody, an anti-Man(α,1-6)Man(α) antibody, an anti-Man(β) antibody, and/or an anti- X(α) antibody. In some embodiments, the kit includes reagents that bind 2, 3, 4, 6, 6, 7, 8, 9, 10, 11, or 12 of these antibodies.

The kit may additionally include a reagent (e.g., a glycan or peptide reagent) that detects an anti-GlcNAc(β,1-4)GlcNAc(β) antibody and/or an anti-Man(α,1-3)Man(α) antibody.

Also provided by the disclosure is a kit useful for differentially diagnosing inflammatory bowel disease (IBD) or non-IBD digestive disease (NIC). The kit includes a reagent (e.g., a glycan or peptide reagent) that detects anti chitobioside (GlcNAc(β,1-4)GlcNAc(β)) carbohydrate antibodies (ACCA) and/or anti-mannan (ASCA) antibodies. The kit may optionally include a reagent that detects anti-laminarobioside (Glc(β,1-3)Glc(β)) Carbohydrate Antibodies (ALCA).

Also disclosed is a method of diagnosing anti-phospholipid syndrome in a subject by providing a test sample from a subject and detecting in the test sample an anti-chitobiose antibody. Levels of the anti- GlcNAc(β, 1-4)GlcNAc(β) antibody in the test sample are compared to the levels of the antibody in a control sample. The control sample may be one or more individuals known to have or not to have anti-phospholipid syndrome. When the control sample has one or more individuals that do not have APS, an elevated level of anti- GlcNAc(β,1-4)GlcNAc(β) antibodies in the test sample as compared to the control sample identifies the subject as having APS.

In some embodiments, the method also includes detecting binding to a β-2 glycoprotein, and comparing the level of binding to the β-2 glycoprotein in the test sample to the level of binding to β-2 glycoprotein in the control sample. Increased binding to the β-2 glycoprotein in the test sample relative to a control sample taken from a non-APS individual or individuals indicates the subject has APS.

The test sample can be, e.g., a biological fluid. Examples of biological fluids include, e.g., whole blood, serum, plasma, spinal cord fluid, urine, or saliva.

The method can optionally include determining the isotype of the antibody. For example the method can include determining whether the antibody is an IgM, IgA, or IgG-type antibody. Also within the disclosure is an array that includes a reagent (preferably a carbohydrate reagent) that specifically detects and anti-GlcNAc(β,1-4)GlcNAc(β) antibody and (optionally) a reagent that detects a β-2 glycoprotein for detecting APS.

The disclosure additionally provides kits for diagnosing APS that include reagents for detecting an anti-chitobiose antibody and (optionally) a β-2 glycoprotein. In some embodiments, the kits contain directions for using the kits to perform the methods described herein.

Any of the kits described herein may be provided with instructions for using the kit. In addition, in some embodiments, the kits are provided with reagents that specifically detect an antibody isotype, e.g., the kit may include one, two, or three reagents that that detect IgA, IgG, IgD or IgM antibodies.

Unless otherwise defined, all technical and scientific terms used herein have the same meaning as commonly understood by a person of ordinary skill in the art to which this invention belongs. In the case of conflict, the present specification, including definitions, will control. In addition, the materials, methods, and examples are illustrative only and not intended to be limiting.

Other features and advantages of the invention will be apparent from the following detailed description and claims.

### BRIEF DESCRIPTION OF THE DRAWINGS

FIG. 1 is a graph showing a Receiver Operator Characteristic (ROC) curve for differentiation between individuals with CD and individuals with other digestive diseases for IgGb3Gb and IgG Mannan antibodies.
FIG. 2A is a box plot graph of the difference between CD colitis and UC groups for the levels of some antiglycan IgG antibodies.
FIG. 2B is a box plot graph of the difference between CD colitis and UC groups for the levels of some antiglycan IgA antibodies.
FIGS. 3A-3C are box plots showing levels of ASCA (IgG), ALCA (IgG) and ACCA (IgA) in CD, UC, and NIC patients.
FIG. 4A is a Receiver Operator Characteristic (ROC) curve of IgA ACCA (GNb4GNb), IgG ALCA (Gb3Gb) and IgG ASCA (Mannan) for differentiating between CD and UC.
FIG. 4B is a Receiver Operator Characteristic (ROC) curve of IgA ACCA (GNb4GNb), IgG ALCA (Gb3Gb) and IgG ASCA (Mannan) for differentiating between NIC and UC.
FIG. 5A is a graph showing the correlation between ASCA IgG and ALCA IgG in CD patients.
FIG. 5B is a graph showing the correlation between ASCA IgG and ACCA IgA in CD patients.
FIG. 5C is a graph showing the correlation between ALCA IgG and ACCA IgA in CD patients.
FIGS. 6A and 6B are histograms showing binding of anti-glycan antibodies to the indicated glycans in sera from patients with anti-phospholipid syndrome (APS).
FIG. 7 is a Receiver Operating Characteristics curve comparing discrimination between CD and UC patients based on levels of ALCA IgG, and ACCA IgA. (CD, Crohn's disease; UC, ulcerative colitis; ALCA, anti-Laminarobioside Carbohydrate Antibodies; ACCA, Anti-Chitobioside Carbohydrate Antibodies).

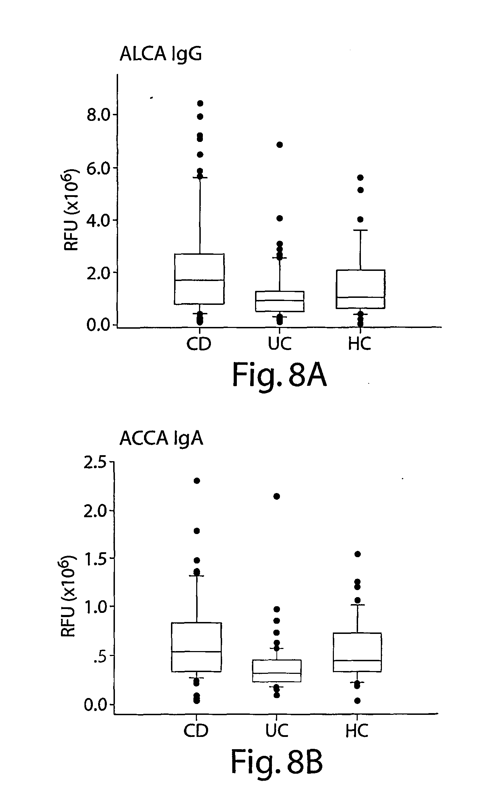

FIGS. 8A and 8B are histograms showing the distribution of ALCA IgG (A) and ACCA IgG (B) antibodies in patients with CD (n=72); UC (n=56) and healthy controls (n=41). Box plots include signals of 50% for the relevant patient population. The median value is indicated inside the box. The lower border represents the 25th percentile, and the upper border represents the 75th percentile. Whiskers above and below the box indicate the 90th and 10th percentiles. CD, Crohn's disease; UC, ulcerative colitis; ALCA, anti-Laminarobioside Carbohydrate Antibodies; ACCA, Anti-Chitobioside Carbohydrate Antibodies.
FIG. 9 is a graph showing the relationship between ALCA, ACCA, and ASCA in the CD cohort tested by presence *versus* absence.

### DETAILED DESCRIPTION OF THE INVENTION

Crohn's disease (CD), irritable bowel syndrome (IBS) and anti-phospholipid syndrome (APS) are diagnosed by examining a test sample from a subject for antibodies to one or more specific glycans. The presence of the antibodies in the test sample identifies the subject as having CD or APS. In some embodiments, elevated levels of glycans in a test sample from the subject as compared to such levels in a reference sample from a subject that does not have CD identifies the test subject as having CD. The methods described herein may be used to distinguish the presence of CD in a subject from other inflammatory bowel diseases (including ulcerative colitis).

A translation of the LinearCode™ syntax used to describe glycan structure to IUPAC nomenclature can be found in Table 1. The glycans are presented either in the International Union of Pure and Applied Chemistry (IUPAC) condensed form for nomenclature carbohydrate representation or in LINEARCODE® syntax, for linear code syntax principles see (Banin et al., Trends in Glycoscience and Glycotechnology, 14:127-37, 2002). Translation of LINEARCODE® to IUPAC representation is in Table 1. All the glycan structures that discussed in this disclosure, unless mentioned otherwise are connected to in the indicated anomericity α or β to other molecular structure, linker, or solid phase.

As used herein, the term "inflammatory bowel disease" is synonymous with "IBD" and is a collective term referring to both Crohn's disease and ulcerative colitis. Thus, an individual having either Crohn's disease or ulcerative colitis is defined herein as having IBD. Conversely, an individual having neither ulcerative colitis nor Crohn's disease does not have IBD as defined herein. The term "inflammatory bowel disease" distinguishes Crohn's disease and ulcerative colitis from all other disorders, syndromes or abnormalities of the gastroenterological tract including irritable bowel syndrome.

As used herein, the term "Non inflammatory bowel disease" is synonymous with "Non-IBD" and is a collective term referring to all other disorders, syndromes or abnormalities of the gastroenterological tract including irritable bowel syndrome (IBS).

The methods for diagnosing IBD may additionally include determining whether a sample is positive for anti-neutrophil cytoplasmic antibodies (ANCA). Anti-neutrophil cytoplasmic antibodies that produce a perinuclear staining pattern (pANCA) are elevated in 60-80% of UC patients and less frequently in CD and other disorders of the colon. Serum titers of ANCA are elevated in UC patients regardless of clinical status and, thus, do not reflect disease activity. High levels of serum ANCA also persist in UC patients five years post-colectomy. Although pANCA is found only very rarely in healthy adults and children, healthy relatives of UC patients have an increased frequency of pANCA, indicating that pANCA may be an immunogenetic susceptibility marker. ANCA reactivity is also present in a small portion of patients with CD. The reported prevalence in CD varies, with most studies reporting that 10 to 30% of CD patients express ANCA (Saxon et al., J. Allergy Clin. Immunol. 86:202-210 (1990); Cambridge et al., Gut 33:668-674 (1992); Pool et al., Gut 3446-50 (1993); and Brokroelofs et al., Dig. Dis. Sci. 39:545-549 (1994)).

As used herein, the term "anti-neutrophil cytoplasmic antibody" is synonymous with "ANCA" and means antibodies to cytoplasmic components of a neutrophil. ANCA, such as serum or saliva ANCA, may be detected using an enzyme-linked immunosorbent assay with alcohol-fixed neutrophils. As disclosed herein, ANCA activity is divided into several broad categories: perinuclear to nuclear staining or cytoplasmic staining with perinuclear highlighting (pANCA); cytoplasmic neutrophil staining without perinuclear highlighting (cANCA); and diffuse staining with speckling across the entire neutrophil (SAPPA). The term ANCA, as used herein, encompasses all varieties of anti-neutrophils cytoplasmic reactivity, including pANCA, cANCA and SAPPA. Similarly, the term "ANCA" encompasses all immunoglobulin isotypes including, for example, immunoglobulin A and G.

The determination of whether a sample is positive for ANCA using non-histological means is made using antigen specific for ANCA using methods described, for example, in US Patent No. 6,218, 129. Antigen specific for ANCA include, for example, whole fixed neutrophils; unpurified or partially purified neutrophil extract; purified UC pANCA antigen such as a purified protein, protein fragment or synthetically produced peptide; anti-ANCA idiotypic antibody; or the like. Particularly useful antigens specific for ANCA are peptides, which can be chemically synthesized or expressed on the surface of phage. Purified antigens specific for ANCA can be, for example, histone H1 or an ANCA-reactive fragment of histone H1, as described, for example, in U.S. Pat. No. 6,074,835; an ulcerative colitis pANCA secretory vesicle antigen or an ANCA-reactive fragment thereof; or a microbial UC pANCA antigen, such as a histone H1-like antigen, porin antigen, Bacteroides antigen, or ANCA-reactive fragment thereof, as described, for example, in U.S. Pat. No. 6,033,864. One skilled in the art understands that additional antigens specific for ANCA, including antigenic fragments and ANCA-reactive peptides, may be identified, for example, using a representative UC pANCA monoclonal antibody.

### Generating an anti-glycan antibody profile

In performing the methods of the invention, a sample to be analyzed has been obtained from the subject to be diagnosed previously i.e. prior to the methods. The term "sample," as used herein, means any biological specimen obtained from an individual that contains antibodies. A sample may be, for example, whole blood, plasma, saliva or other bodily fluid or tissue having antibodies, preferably a serum sample. Samples may be diluted, if desired, before they are analyzed for anti-glycan antibodies. The subject may be, e.g., a human, non-human primate (including a chimpanzee, ape, gorilla, old world primate), cow, horse, dog, cat, pig, goat, sheep, or rodent (including, e.g., a mouse, rat, or guinea pig). Anti-glycan profiles may be determined using methods known in the art for identifying antibodies to glycans including those disclosed in e.g., WO00/49412, WO02/064556, or Schwarz et al., Glycobiology 13:749-54, 2003.

These methods are typically performed using reagents that specifically bind to the anti-glycan antibodies. The reagents may be, e.g., the specific glycan structures. Alternatively, the reagents may be other molecules or macromolecules that include the specific glycan structure. For example, the anti-Glc(β,1-3)Glc(β) antibody may be detected using the polysaccharide β-D(1-3)Glucan, a polymer of glucose units connected in a (β,1-3)glycosidic bond. Thus, the glycan itself may be used for detecting the corresponding antibody or antibodies, as can any carbohydrate, peptide, protein, or any other molecular structure that includes the glycan.

If desired, the peptides that mimic carbohydrate antigens may be used in the methods and compositions described herein. The peptides may be used to identify specific anti-glycan antibodies. Peptides which mimic structures recognized by antiglycan antibodies may be identified using methods known in the art, e.g., by screening a filamentous phage-displayed random peptide library (Zhan et al., Biochem Biophys Res Commun. 308:19-22,2003; Hou et al., J Immunol. 17:4373-79, 2003).

Glycan antigens used to identify various anti-glycan antibodies may be obtained from a variety of other sources so long as the antigen is capable of binding specifically to the given anti-glycan. Binding to anti-glycan antibodies may be performed using variety of other immunoassay formats known in the art. For example, competitive and non-competitive immunoassay formats may be used (Self and Cook, Curr. Opin. Biotechnol. 7:60-65 (1996)).

Other assays include immunoassays, such as enzyme-linked immunosorbent assays (ELISAs). An enzyme such as horseradish peroxidase (HRP), alkaline phosphatase (AP), β-galactosidase or urease may be linked to a secondary antibody selective for a primary anti-glycan antibody of interest. A horseradish-peroxidase detection system may be used, for example, with the chromogenic substrate tetramethylbenzidine (TMB), which yields a soluble product in the presence of hydrogen peroxide that is detectable at 450 nm. An alkaline phosphatase detection system may be used with the chromogenic substrate p-nitrophenyl phosphate, for example, which yields a soluble product readily detectable at 405 nm. Similarly, a β-galactosidase detection system may be used with the chromogenic substrate o-nitrophenyl- a β-D-galactopyranoside (ONPG), which yields a soluble product detectable at 410 nm, or a urease detection system can be used with a substrate such as urea-bromocresol purple (Sigma Immunochemicals, St. Louis, Mo.). A useful secondary antibody linked to an enzyme may be obtained from a number of commercial sources; goat F(ab')₂ anti-human IgG-alkaline phosphatase, for example, may be purchased from Jackson Immuno-Research (West Grove, Pa.).

Immunoassays encompass capillary electrophoresis based immunoassays (CEIA) and may be automated, if desired. Immunoassays may also be used in conjunction with laser-induced fluorescence (see, for example, Schmalzing and Nashabeh, Electrophoresis 18:2184-93 (1997)); Bao, J. Chromatogr. B. Biomed. Sci. 699:463-80 (1997)).

Liposome immunoassays, such as flow-injection liposome immunoassays and liposome immunosensors, may also be used (Rongen et al., J. Immunol. Methods 204:105-133 (1997)).

A radioimmunoassay may also be used for determining whether a sample is positive for a glycan antibody or for determining the level of anti-glycan antibodies in a sample. A radioimmunoassay using, for example, an ¹²⁵Iodine- labeled secondary antibody (Harlow and Lane, Antibodies A Laboratory Manual Cold Spring Harbor Laboratory: New York, 1988) is encompassed within the invention.

A secondary antibody may alternatively be labeled with a chemiluminescent marker. Such a chemiluminescent secondary antibody is convenient for sensitive, non-radioactive detection of anti-glycan antibodies and may be obtained commercially from various sources, such as Amersham Lifesciences, Inc. (Arlington Heights, III.).

A detectable reagent may also be labeled with a fluorochrome. Appropriate fluorochromes include, for example, DAPI, fluorescein, Hoechst. 33258, R-phycocyanin, B-phycoerythrin, R-phycoerythrin, rhodamine, Texas red or lissamine. A particularly useful fluorochrome is fluorescein or rhodamine. Secondary antibodies linked to fluorochromes can be obtained commercially. For example, goat F(ab')₂ anti-human IgG-FITC is available from Tago Immunologicals (Burlingame, Calif.).

A signal from the detectable reagent may be analyzed, for example, using a spectrophotometer to detect color from a chromogenic substrate; a radiation counter to detect radiation, such as a gamma counter for detection of ¹²⁵Iodine; or a fluorometer to detect fluorescence in the presence of light of a certain wavelength. For detection of enzyme-linked reagents, a quantitative analysis of the amount of anti-glycan antibodies can be made using a spectrophotometer such as an EMAX Microplate Reader (Molecular Devices, Menlo Park, Calif.) in accordance with the manufacturer's instructions. If desired, the assays of the invention may be automated or performed robotically, and the signal from multiple samples may be detected simultaneously.

Other methods include, e.g., flow cytometry (including bead based immunoassays) and phage display technology for expressing a recombinant antigen specific for an anti-glycan antibody. Phage particles expressing the antigen specific for a desired anti-glycan antibody can be anchored, if desired, to a multiwell plate using an antibody such as an anti phage monoclonal antibody (Felici et al., "Phage-Displayed Peptides as Tools for Characterization of Human Sera" in Abelson (Ed.), Methods in Enzymol. 267, San Diego: Academic Press, Inc. (1996)).

Anti-glycan antibodies may be conveniently detected by simultaneously analyzing multiple samples for the presence of one or more anti-glycan antibodies. For example, the antibodies may be detected using an array of reagents that can bind specifically to the anti-glycan antibodies. Preferably, each reagent is provided in a different location with a defined address on the array. By exposing the sample to this array, all the anti glycan antibodies that bind to the reagent on the array may be detected in one test. Suitable arrays that include reagents (preferably carbohydrate reagents) that specifically detect the CD-detecting antibodies disclosed herein, e.g., an anti-Glc(β) antibody, an anti-Glc(β,1-4)Glc(β) antibody, an anti-Glc(β,1-3)Glc(β) antibody, an anti-GlcNAc(β) 6-sulfate antibody, an anti-Man(α,1-2)Man(α) antibody, an anti-Man(α,1-3)Man(α) antibody, an anti-Man(α,1-6)Man(α) antibody, an anti-Man(α) antibody, an anti-Man(α,1-3)[Man(α,1-6)]Man(α), an anti-Manna antibody, an anti-Dextran antibody, an anti- Xylan antibody, an anti-GlcNAc(β,1-4)GlcNAc(β) antibody, an anti-Gal 3-sulphate(β) antibody, an anti-aGlcNAc(β,1-3)GalNAc(β) antibody, an anti-GlcNAc(β,1-3)Gal(β,1-4)Glc(β)antibody, an anti-Gal(α) antibody, an anti-Gal(β) antibody, an anti- GalNAc(α), an anti-Glc(α) antibody, an anti-Gal(β,1-6)Gal(β) antibody, an anti anti-GlcNAc(β,1-6)GalNAc(α) or an anti-Gal(α,1-3)Gal(β,1-4)GlcNAc(β) antibody for diagnosing CD.

In some embodiments, the reagents that are used to specifically bind and detect those anti glycans antibodies are the specific glycan structures. In other embodiments, the reagents are other molecules or macromolecules that include the specific glycan structure. For example, the anti-Glc(β,1-3)Glc(β) antibody may be detected using the polysaccharide β-D(1-3)Glucan, a polymer of glucose units connected in a (β,1-3)glycosidic bond. Thus, the glycan itself may be used for detecting the corresponding antibody or antibodies, as can any carbohydrate, peptide, protein, or any other molecular structure that includes the glycan.

The array may additionally include a reagent or reagents, e.g., a carbohydrate reagent or reagents, that detect an anti-Mannan antibodies or a ANCA. In some embodiments, the glycans are attached to the array via a linker. A suitable linker includes at least one ethylene glycol derivative, at least two cyanuric chloride derivatives and an anilino group.

Arrays useful for diagnosing APD is a reagent (preferably a carbohydrate reagent) that specifically detects an anti-chitobiose antibody and, optionally, a reagent that specifically detects a β-2 glycoprotein.

If desired, peptides that mimic carbohydrate antigens may be used in the methods and compositions described herein. The peptides may be used to identify specific anti-glycan antibodies. Peptides which mimic structures recognized by antiglycan antibodies may be identified using methods known in the art, e.g., by screening a filamentous phage-displayed random peptide library (Zhan et al., Biochem Biophys Res Commun. 308:19-22,2003; Hou et al., J Immunol. 17:4373-79, 2003).

### Interpreting anti-glycan antibody binding data

Typically, binding of anti-glycan antibodies to glycans in a sample is compared to a reference population, and differences in levels of the anti-glycan antibodies in the two samples are compared. The threshold for determining whether a test sample is scored positive for CD or APS , or Non-IBD based on its ant-glycan antibody profile may be altered depending on the sensitivity or specificity desired. The clinical parameters of sensitivity, specificity, negative predictive value, positive predictive value, and overall agreement are calculated using true positives, false positives, false negatives and true negatives. A "true positive" sample is a sample positive for CD according to colonoscopy, radiologic and/or histologic analysis, which is also diagnosed positive according to a method of the invention. A "false positive" sample is a sample negative for CD by colonoscopic, radiologic and/or histologic analysis, which is diagnosed positive according to a method of the invention. Similarly, a "false negative" is a sample positive for CD by colonoscopic, radiologic and/or histologic analysis, which is diagnosed negative according to a method of the invention. A "true negative" is a sample negative for CD by colonoscopic, radiologic and/or histologic analysis, and also negative for CD according to a method of the invention. see, for example, Mousy (Ed.), Intuitive Biostatistics New York: Oxford University Press (1995).

As used herein, the term "sensitivity" means the probability that a laboratory method is positive in the presence of CD. Sensitivity is calculated as the number of true positive results divided by the sum of the true positives and false negatives. Sensitivity essentially is a measure of how well a method correctly identifies those with disease. In a method of the invention, the anti-glycan antibody values may be selected such that the sensitivity of diagnosing an individual is at least about 60%, and can be, for example, at least about 65%, 70%, 75%, 80%, 85%, 90% or 95%.

As used herein, the term "specificity" means the probability that a method is negative in the absence of CD. Specificity is calculated as the number of true negative results divided by the sum of the true negatives and false positives. Specificity is essentially a measure of how well a method excludes those who do not have CD. The anti-glycan cut-off value can be selected such that, when the sensitivity is at least about 70%, the specificity of diagnosing an individual is in the range of 30-60%, for example, 35-60%, 40-60%, 45-60% or 50-60%.

The term "positive predictive value," as used herein, is synonymous with "PPV" and means the probability that an individual diagnosed as having CD actually has the disease. Positive predictive value can be calculated as the number of true positives divided by the sum of the true positives and false positives. Positive predictive value is determined by the characteristics of the diagnostic method as well as the prevalence of the disease in the population analyzed. In a method of the invention, the anti-glycan antibody cut-off values may be selected such that the positive predictive value of the method in a population having a CD disease prevalence of 15% is at least about 5%, and may be, for example, at least about 8%, 10%, 15%, 20%, 25%, 30% or 40%.

As used herein, the term "overall agreement" means the accuracy with which a method diagnoses a disease state. Overall agreement is calculated as the sum of the true positives and true negatives divided by the total number of sample results and is affected by the prevalence of CD in the population analyzed. The anti-glycan antibody cut-off values may be selected such that the overall agreement of a method of the invention in a patient population having a CD disease prevalence of 15% is at least about 45%, and may be, for example, at least about 50%, 55% or 60%.

The invention will be illustrated in the following non-limiting examples.

### Example 1. Comparative antiglycan antibody levels in the serum of Crohn's Disease patients and patients with other digestive diseases.

An anti-glycan antibody profile for IgG, IgA and IgM in the serum of the patients was obtained using GlycoChip® arrays (Glycominds, Ltd., Lod, Israel, Cat No. 9100). The arrays were constructed using procedures described in Schwarz et. al. Glycobiology, 13: 749-54, 2003. Anti-glycan antibody profiles of 45 CD patients and 27 patients with other digestive diseases were compared.

All serum samples were tested using GlycoChip® plates (Glycominds Ltd., Lod , Israel, Cat No. 9100), which are arrays of mono- and oligosaccharides covalently attached to a reduced volume 384-well micro titer plate. The mono- and oligosaccharides displayed on the array are listed in Table 1. A translation of the LinearCode™ syntax used to describe glycan structure to IUPAC nomenclature is found in Table 1.

The sera from patients volunteers who had signed an informed consent form were collected by Dr. Iris Dotan from the Gastroenterology and Liver Disease Institute in the Tel Aviv Sorasky Medical Center, Israel. All patients were diagnosed by Dr. Iris Dotan. The sera were collected in evacuated silicon coated gel containing tubes (Estar Technologies Cat# 616603GLV). The sera were separated from the blood cells and kept frozen at -25° C until use. The volume of all solutions added to the glycan array was 10µl/well. The sera were diluted (1:20; saturating concentration) in 0.15M Tris-HCl pH 7.2, 0.085M Mg2S04, 0.05% Tween 20 (TBST) containing 1% BSA (Sigma), dispensed into glycan array plates using a Tecan Genesis Workstation 200 automated handling system, and incubated for 60min at 37°C. The plates were then washed with 250 µL/well Phosphate buffered Saline with 0.05 % Tween 20 (PBST, Sigma) in an automatic plate washer (Tecan, POWERWASHER™). At this point, the following reagents, diluted in TBST with 1% BSA, were added using a Multidrop 384 dispenser (Thermo Labsystems) and incubated for 60 min at 37°C: for IgG, IgA, and IgM determination - the respective sub-class specific biotinylated goat anti- human Ig antibody (Jackson, PA, USA) at 2.8µg/ml, 3µg/ml, and 0.9µg/ml, respectively. Following washing with PBST, Streptavidin-conjugated europium (0.1 µg/ml) diluted in TBST with 1% BSA was added to each well followed by incubation for 30 min at 37°C in the dark, and washing with PBST. DELFIA™ enhancement solution was then added to the wells and the plates were incubated for 30 to 45 min in the dark at room temperature. The fluorescence of the wells was read with a Victor 1420 (Wallac, Finland) plate reader using time resolved fluorescence settings of 340/612 nm (Excitation/Emission).

Some patients were tested for the presence of antibodies to perinuclear anti neutrophil cytoplasmic antibodies (pANCA) and anti-Saccharomyces cerevisiae (ASCA) IgG and IgA using a commercial kits made by INOVA, San-Diego, CA Cat. No 708290, 708865, 708870 respectively, according to the manufacturer instructions.

Tables 2, 3 and 4 present levels of IgG, IgA and IgM type antiglycan antibodies that were detected at significantly different levels between the CD population and the population with other digestive diseases. The tables show fluorescent signals from binding of IgG antibodies to different glycans in CD patients and non CD patients. Glycans are presented in LINEARCODE®. The values presented for IgG and IgA are absolute values. The values presented for IgM are absolute values after reduction of background. The back ground signal was measured as the signal received from wells with covalently bound p-nithrophenol. If the result was negative the signal was scored as zero.

Comparison of the average and median values of anti-carbohydrate antibodies in the CD and other digestive disease populations reveals a significant elevation in most of the anti-glycans antibodies in the CD group as compared to the group containing individuals with the other digestive diseases group. None of the CD patients was found to be positive for pANCA antibodies. All the anti-glycans levels that are displayed in Tables 2 , 3 and 4 show statistically significant (α=0.05; p<0.05) differences between the CD groups and the other digestive disease or normal group. Statistically significant differences between the medians of signals of CD and other digestive disease population and normal population were observed for antibodies bound to the following glycans: Glc(β), Glc(β,1-4)Glc(β), Glc(β,1-3)Glc(β), GlcNAc(β) 6-sulfate, Man(α,1-2)Man(α), Man(α,1-3)Man(α), Man(α,1-6)Man(α), Man(α), Man(α,1-3) [Man(α,1-6)]Man(a), Mannan, Dextran, Xylan, GlcNAc(β,1-4)GlcNAc(β), Gal 3-sulphate(β), GlcNAc(β,1-3)GalNAc(β), GlcNAc(β,1-3)Gal(β,1-4)Glc(β), Gal(α), Gal(β), GalNAc(α), Glc(α), Gal(β,1-6)Gal(β), GlcNAc(β,1-6)GalNAc(α) and Gal(α,1-3)Gal(β,1-4)GlcNAc(β).

Table 5 shows the specificity and sensitivity of the different IgG anti glycans for differentiation between CD and other digestive diseases using different cut-off values. The cutoff values for each glycans where set as the 89^{th} percentile of the non CD group.

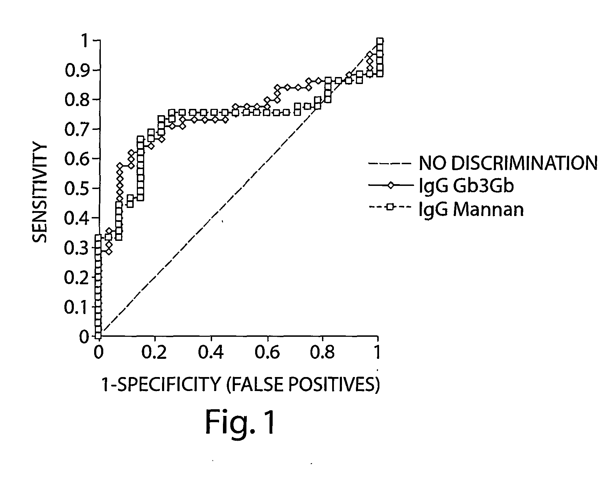

These results reveal a set of chemically defined glycan antigens that are useful for diagnosing CD. The levels of antibodies to those glycans are higher in the CD population than in the population of normal individuals or individuals with other digestive diseases. The antibodies that showed the greatest differentiation between CD and other digestive diseases in these studies are a set of antibodies to mannose based glycan fragment as well as antibodies to on Glc(β), Glc(β,1-4) Glc(β), Glc(β,1-3)Glc(β). Antibodies to Glc(β,1-3)Glc(β), Man(α,1-3)Man(α) and Man(α,1-3)[Man (α 1-6)]Man(α) were in particular able to differentiate between CD and other digestive disease at 57-62% sensitivity and 89%-93% specificity. The separation of those structures was better that what was achieved with Mannan (ASCA) 47% sensitivity and 89% specificity. Table 6 demonstrates that it is possible to use different cut of levels and to achieve higher sensitivity but lower specificity. Table 6 describes the sensitivity, specificity, True Positives (TP), True Negative (TN), False Positives (FP), and False Negatives (FN) and Positive Predictive value (PPV) in different cut-of value for differentiation between CD and other digestive disease according to the level of Anti Glc(β,1-3)Glc (β), IgG and anti Mannan IgG. FIG. 1 is a Receiver Operator Characteristic (ROC) curve for differentiation between individuals with CD and individuals with other digestive diseases according to levels of anti Glc(β,1-3)Glc(β), IgG and anti Manna IgG antibodies.

By using combination of two or more glycans it is possible to improve the sensitivity with without reducing the specificity. For example, by setting cut-offs of 2000,000 for anti-Glc(β,1-3)Glc(β) and 2,400,000 for anti-Mannan and setting the criteria for identification of CD as those individuals who are above cut-off levels for either of the antibodies it is possible to achieve 82% sensitivity with 70% specificity. Achieving this sensitivity by each of the antibodies alone would require lower cut off points, but these lower cutoffs would lead to poor specificity (e.g., a specificity of 37% for Glc(β,1-3)Glc(β)).

### Example 2. Comparative antiglycan antibody levels in the serum of Crohn's Disease (CD) Colitis patients and Ulcerative Colitis (UC) patients.

An anti-glycan antibody profile for IgG and IgA in the serum of the patients was obtained using GlycoChip® arrays (Glycominds, Ltd., Lod, Israel, Cat No. 9100). The arrays were constructed using procedures described in Schwarz et. al., Glycobiology 13: 749-54, 2003. Anti-glycan antibody profiles of 6 CD colitis patients and 19 UC patients were compared. All serum samples were collected and tested as described in Example 1.

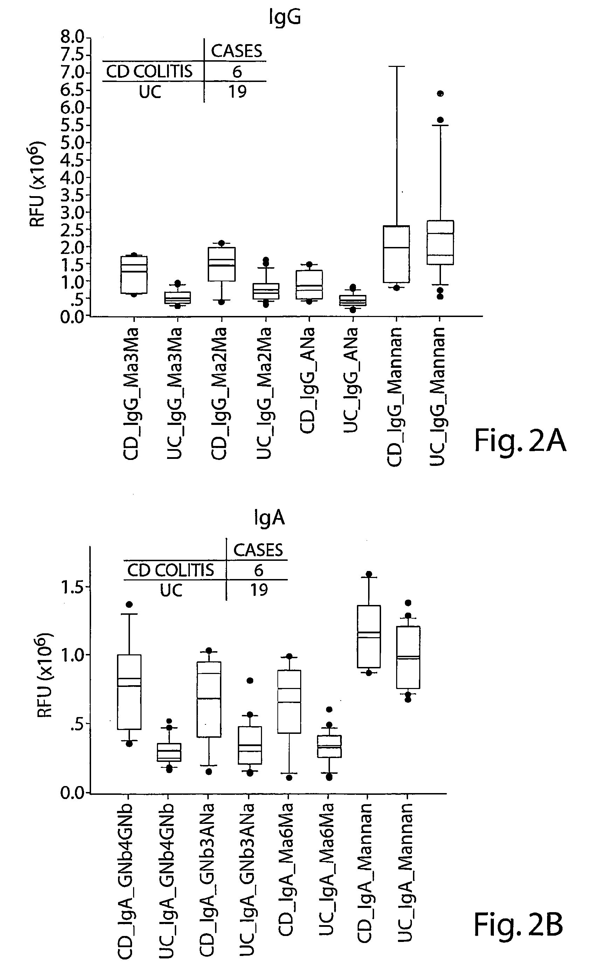

Tables 7 and 8 show the levels of IgG and IgA-type antiglycan antibodies, respectively, that were detected at significantly different levels between the CD Colitis population and the UC population. Glycans are presented in LINEARCODE®. The values presented for IgG and IgA are absolute values. Comparison of the average and median values of anti-carbohydrate antibodies in the CD colitis patients and UC patients populations reveals a significant elevation in most of the anti glycans antibodies in the CD group as compared to the group containing individuals with the other digestive diseases group. All the anti glycans levels that are displayed in Tables 7 and 8 show statistically significant (α=0.05; p<0.05) differences between the CD Colitis group and the UC group, with the exception of anti Mannan (ASCA) IgA and IgG. The most significant difference between the antibodies levels in the IgG class was found in the levels of anti Man(α,1-3)Man(α), whereas for the IgA class the most significant difference was found between the levels of anti GlcNAc(β,1-4)GlcNAc(β) antibodies. No statistically significant difference between the levels of anti Mannan (IgG or IgA) levels of the CD Colitis patients and UC patients populations was detected in these studies. FIG. 2 is a box plot graph of the difference between CD colitis and UC groups for the levels of some antiglycan IgG and IgA antibodies.

### Example 3. Comparative antiglycan antibody levels in the serum of Crohn's Disease (CD) patients, Ulcerative Colitis (UC) patients, and Irritable Bowel Syndrome (IBS) patients.

The levels of antiglycan antibodies in serum from CD, UC, and IBS patients were compared.

An anti-glycan antibody profile for IgG, and IgA in the serum of the patients was obtained using GlycoChip® arrays (Glycominds, Ltd., Lod, Israel, Cat No. 9100). The arrays were constructed using procedures described in Schwarz et. al. Glycobiology 13:749-54, 2003. The levels of the following Anti-glycan antibody were measured: Anti Laminarobioside (Glc(β,1-3)Glc(β)) Carbohydrate Antibodies (ALCA); Anti Chitobioside (GlcNAc(β,1-4)GlcNAc(β)) Carbohydrate Antibodies (ACCA); and Anti mannan (Anti Saccromyces cerevisiae Antigen (ASCA)). Those antibodies were measured in the serum 70 CD patients, 56 UC patients and 19 patients with Non-IBD digestive diseases Controls (NIC) were also compared. All serum samples were collected and tested using GlycoChip® plates (Glycominds Ltd., Lod, Israel, Cat No. 9100) as described in Example 1.

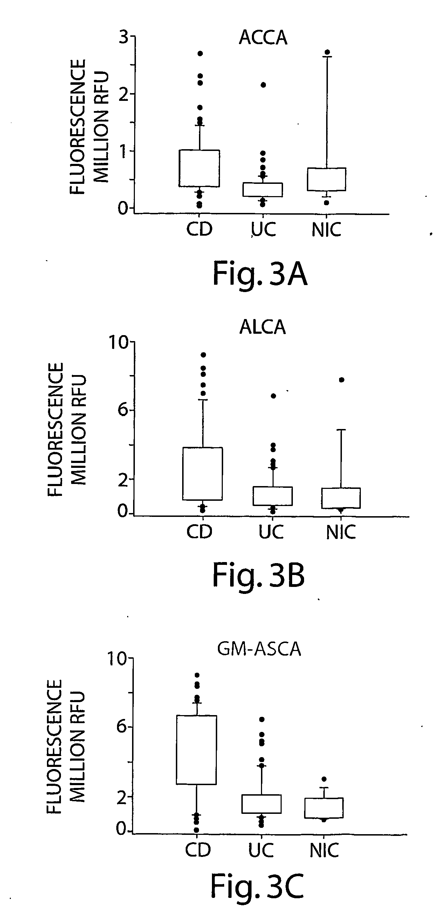

A summary of the results is presented in FIG. 3. CD patients have statistically significant higher levels of ASCA, ALCA and ACCA then UC patients. UC patients had statistically significant lower levels of ASCA, ALCA and ACCA than NIC patients. NIC patients had statistically significant higher IgA ACCA levels then UC patients.

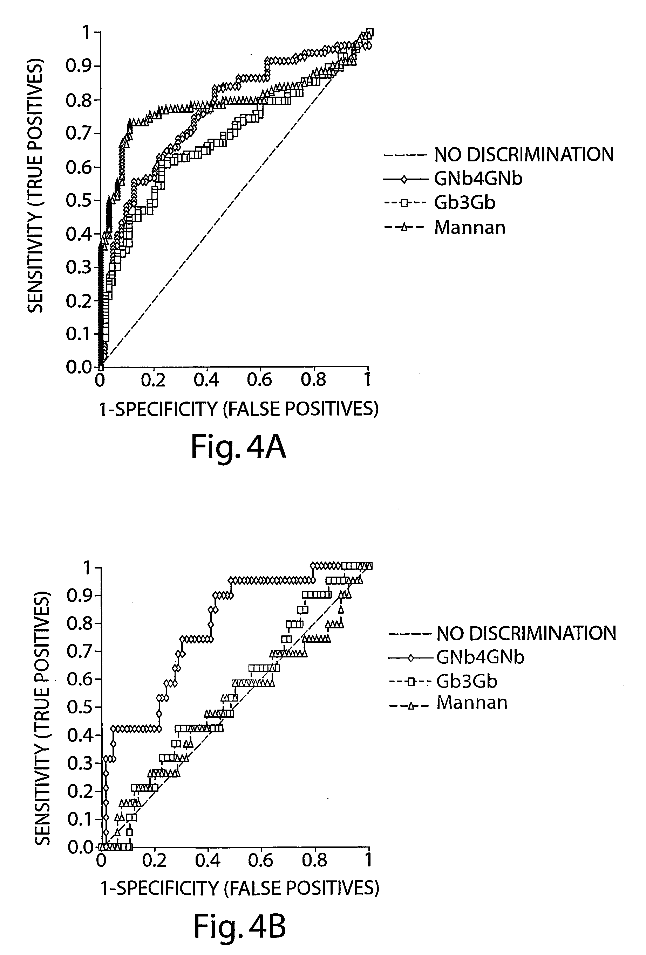

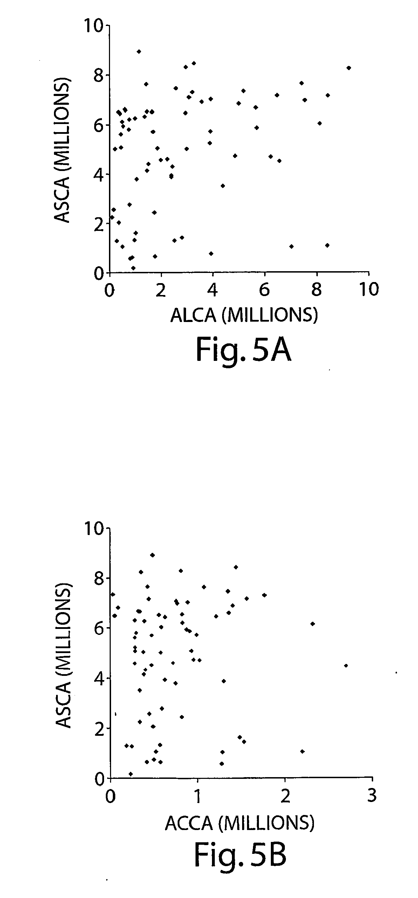

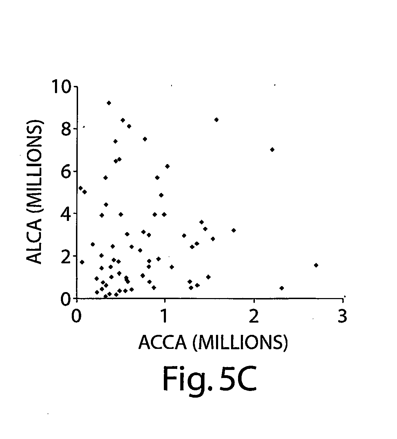

The ROC curve in FIG. 4A demonstrates that all three markers can be used to differentiate between CD and UC. The ROC curve in FIG. 4B demonstrates that ACCA allows for differentiation between NIC and UC and shows the sensitivity and specificity for different cutoff levels. FIGS. 5A-C below show low or no correlation between the levels of IgG ASCA marker and IgG ALCA or IgA ACCA in CD patients. It is clear that each marker recognizes distinct sub-groups of Crohn's Disease patients.

Table 9 summarizes the sensitivity and specificity for each disease type using the combinations of the markers: a sample that tests ASCA(+) or ALCA (+) indicates the patient from whom the sample was obtained has CD and not UC. A patient who tests IgA ACCA(+) and ASCA(-) has a non-IBD digestive disease.

These data demonstrate that ASCA, ACCA, and ALCA-markers may be used to differentially diagnose IBD (which includes CD and IBD) from all other intestinal diseases (NIC), including irritable bowel syndrome (IBS). The presence of antibodies to IgA ACCA and the absence of antibodies to ASCA indicates a subject has NIC. A subject is also diagnosed with NIC if ALCA antibodies are also absent from the sample.

### Example 4. Levels of antiglycan antibody levels in the serum of Anti-Phospholipid Syndrome (APS) patients and patients with other digestive diseases.

A pool of serum samples from APS patients was fractionated on a β-2 glycoprotein column and tested for the presence of anti-glycan antibodies

Antibody binding was examined using GLYCOCHIP™ substrates as described in WO00/49412. Wells were blocked with ddH₂0/BSA 2.5%. The serum sample was diluted 1:2 in 1% TBST/BSA. Anti-IgA, IgG, and IgM samples were diluted 1:100 in TBST/BSA 1%. The Alexa 633 dye (Molecular Probes, Eugene, OR, # S-20992) was diluted 1:150 in TBST 1:150. Samples were injected using a Tescan HS4800 program. Dry arrays were scanned using an Affymetrix 428 Scanner, and images were analyzed using 'ArrayPro' software. Numerical values were exported to Excel and analyzed. For isotype determination, anti-human IgG, Fc gamma fragment specific\Biotin (Goat); Jackson; Cat # 109-065-008, anti-human IgM, Fc 5mu fragment specific\Biotin (Goat); Jackson; Cat # 109-065-043, and anti-human serum IgA\biotin (Goat); Jackson; Cat # 109-065-011.

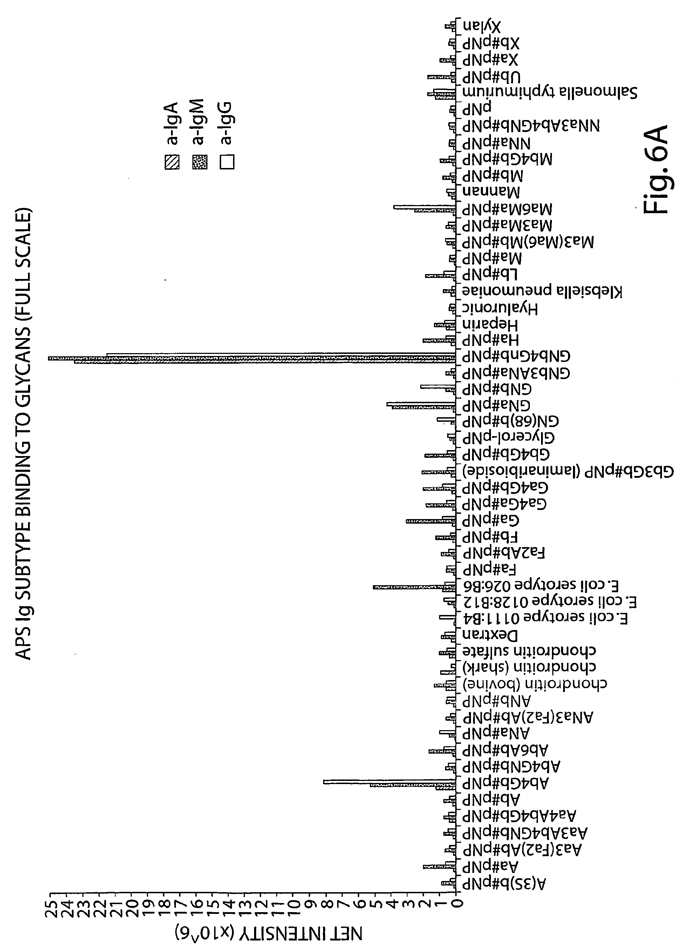

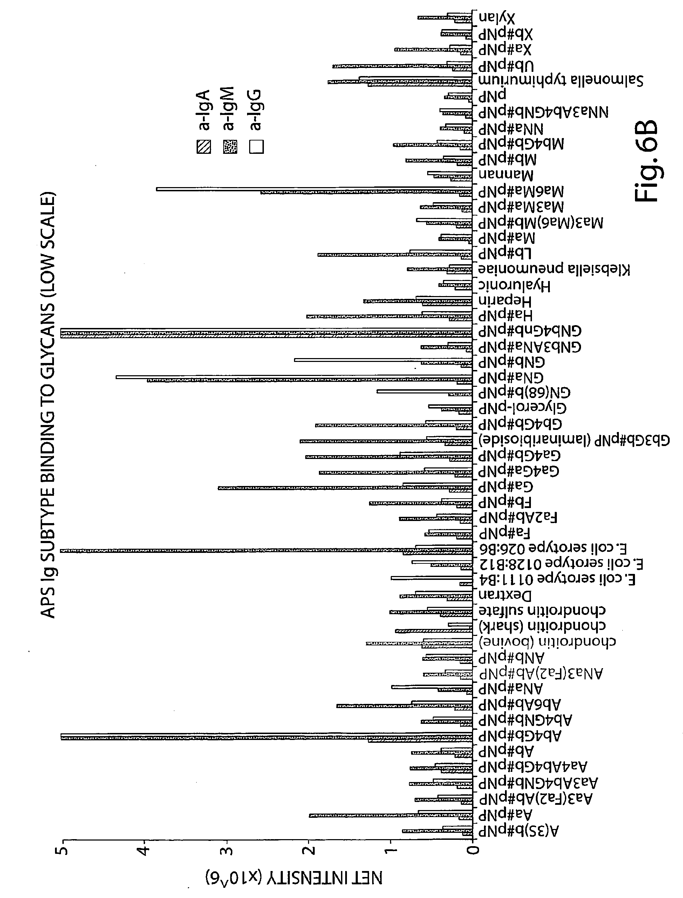

The serum sample was affinity-purified against a column of β-2-glycoprotein and then applied to a GlycoChip containing multiple glycans. The full magnitude (0-2.5x10⁷) of the interaction profile of the APS immunoglobulins and the tested glycans antibodies on the GlycoChip are shown in FIG. 6A. A smaller scale (0-5x10⁶) of the binding is shown in FIG. 6B.

The highest levels of antibodies were observed for antibodies against GlcNAc(β,1-4)GlcNAc(β) for each of the IgA, IgG, and IGM subclasses High IgG levels against LPS from Salmonella typhimurium, Man(α,1-6)Man(α), GlcNAc(α), GlcNAc(β) and Gal(β,1-4)Glc(β) (Lactose) were also observed.

IgA levels were highest against GlcNAc(β,1-4)GlcNAc(β) and relatively high against Gal(β,1-4)Glc(β) and LPS from Salmonella. IgM levels were highest against GlcNAc(β,1-4)GlcNAc(β) and relatively high against LPS from E. coli O26:B6, Glucose derivatives such as Glc(α), Glc(α,1-4)Glc(α), Glc(α,1-4)Glc(β), Glc(β,1-3)Glc(β) and Glc(β,1-4)Glc(β), GlcNAc(α), Rha(α) as well as Man(α,1-6)Man(α), GalA(β), GlcA(β) and LPS from Salmonella. Relatively low levels of Ig were detected against the preparation of Mannan used on the GlycoChip substrate.

These results demonstrate that elevated levels antibodies to GlcNAc(β,1-4)GlcNAc(β) in the blood may serve as a marker for diagnosis of APS, and/or for the severity of the disease.

### Example 5. Serum antibodies against laminarobioside and chitobioside are markers of Crohn's disease

To investigate anti-glycan antibodies as potential serological markers in IBD, an array of sugars (mono-, di- and tri-saccharides) were used to characterize the anti-glycan antibody profile in serum samples from CD patients, UC patients and healthy blood donors.

### Materials and Methods

### Patients

Signed informed consents were obtained from all patients, and the study was approved by a local Ethical Committee. Patients with IBD were identified based on accepted clinical, radiological, endoscopic and histological criteria (Table 10). Blood samples were obtained in the Department of Gastroenterology and Liver Diseases, Tel Aviv Sourasky Medical Center, and were collected in evacuated silicon-coated tubes containing gel (Estar Technologies, Israel). After blood coagulation, serum was separated by centrifugation, collected and kept frozen at -25°C until use. The laboratory evaluations were conducted in a blinded manner.

### Profiling anti-glycan antibodies using glycan array

Profiling of anti-glycan antibodies in all serum samples was done using a GLYCOCHIP^{®} array, a glycan array in the 384 wells microtiter plate format as described (Schwarz et al., Glycobiology 13:749-54, 2003). Briefly, the p-nitrophenyl saccharides were covalently bound to the surface with a linker (oligomer of 1,8-diamino-3,6-dioxaoctan; Sigma, St. Louis, MO). Polysaccharides tested are detailed in Table 12.

The volume of all solutions added to the glycan array was 10 µL/well. The sera were diluted 1:40 in 0.15 M Tris-HCl pH 7.2, 0.085 M Mg₂SO₄, 0.05% Tween-20 (TBST) containing 1% bovine serum albumin (BSA, Sigma, MO, USA), dispensed into glycan array chips using a Tecan Genesis Workstation 200 automated handling system (Tecan, Switzerland), and incubated for 60 min at 37°C. The plates were then washed with 250 µL/well phosphate-buffered saline (PBS) with 0.05% Tween-20 (PBST, Sigma) in an automatic plate washer (PowerWasher™, Tecan). At this point, for IgG, IgA and IgM determination, the respective subclass-specific biotinylated goat anti-human Ig antibody (Jackson Laboratories, PA, USA) diluted in TBST with 1% BSA, was added and plates incubated for 60 min at 37°C. Following washing with PBST, streptavidin-conjugated europium (Perkin-Elmer, CA, USA, 0.1 µg/mL) diluted in TBST with 1% BSA was added to each well, followed by incubation for 30 min at 37°C in the dark and washing with PBST. Delfia™ (Perkin-Elmer). Enhancement solution was then added to the wells and the chips were incubated for 30-45 min in the dark at room temperature. The fluorescence of the wells was read with a Victor™ 1420 (Perkin-Elmer) multi-label counter using time-resolved fluorescence settings of 340/612 nm (excitation/emission).

### Determination IgG ALCA positive and IgA ACCA positive patients

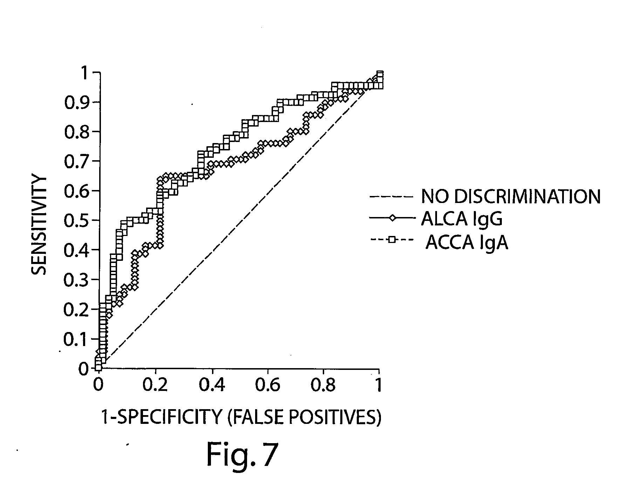

ALCA IgG and ACCA IgA reactivities were determined using 2.5*10⁶ relative fluorescence units (RFU) and 0.74*10⁶ RFU cutoff values, respectively. These cut-off values were determined using receiver operating characteristic (ROC) curves (FIG. 7) to provide optimal positive and negative predictive values for differentiation between CD and UC patients (ACCA IgA, 38% sensitivity, 95% specificity: ALCA IgG, 28% sensitivity, 91 % specificity).

### Determination of IgG ASCA

Serum levels of IgG ASCA were determined by the QUANTA LiteTM ASCA (*S*. *cerevisiae*) IgG kit (Inova Diagnostics Inc., Calif. U.S.A) according to the manuacturer's protocol. The results were presented in arbitrary units and were calculated by dividing the average optical density of the sample by the average optical density of the ASCA IgG ELISA Low Positive. The result is multiplied by the number of units assigned to the ASCA IgG ELISA Low Positive. Results were expressed as negative (<20 U), equivocal (between 20 and 25 U) or positive (> 25 U).

### Statistical Analysis

Descriptive statistics were used for population and subgroup characteristics. For comparison between the study groups (diseases and control), one-way ANOVA was used were a p-value < 0.05 was considered to be statistically significant.

For results obtained using the GlycoChip^{®}, ROC curves, generated by plotting sensitivity *versus* 1-specificity, were calculated for GlycoChip^{®} data. The curves were used to determine cutoff values providing optimal positive and negative predictive values.

### Results

### Patient characteristics

Serum samples were obtained from CD patients (n=72), UC patients (n=56), and healthy blood donors (n=41). UC patients after restorative proctocolectomy and ileal-pouch-anal anastomosis were excluded. There were no significant differences in demographic parameters between the groups studied (Table 10). Bowel operations were significantly more frequent in the CD *versus* UC group (p<0.0001), and the use of 5-ASA (*p*=0.0001) and biological treatments (Infilixmab, *p*=0.049) was significantly higher in the CD group.

### Anti-glycan antibodies in CD patients, UC patients and healthy controls

Serum samples were initially screened for 49 different glycans (Table 11). Antibodies against mannan and mannan residues, and 5 IgG and 2 IgA anti-glycan antibodies were significantly elevated in CD *versus* the UC patients (*p*<0.001) (Tables 12-13). Anti-Glc(β1,3)Glc(β) (Laminarobioside) IgG and anti-GlcNAc(β1,4)GlcNAc(β) (Chitobioside) IgA were the most prominent antibodies, demonstrating the highest mean RFU in the CD group, and the most significant *p*-values between CD and UC patients. Anti-Glc(β1,3)Glc(β) (Laminarobioside) IgG in was also significantly higher in CD patients *versus* healthy controls (*p*=0.05), while anti-GlcNAc(β1,4)GlcNAc(β) (Chitobioside) IgA was comparable between the CD patients and healthy controls (*p*=0.15). These antibodies were thus designated ALCA =Anti-Laminarobioside Carbohydrate Antibodies and ACCA=Anti-Chitobiose Carbohydrate Antibodies. The distribution of the ALCA IgG and ACCA IgA in the CD patients, UC patients and healthy controls is described in FIG. 8. Binding specificities of ALCA IgG and ACCA IgA were verified by inhibition studies using an soluble antigen.

ROC curves describing the ability of ALCA IgG, ACCA IgA, and ASCA IgG to discriminate between CD and UC patients are shown in FIG. 2.

No anti-glycan antibodies were elevated in the UC *versus* CD patients or healthy controls. There were no significantly elevated anti-glycan IgM antibodies in the groups studied.

### Correlation between antibodies against mannan using a glycan array and a commercial ASCA assay

To compare readouts from the GlycoChip^{®} with a validated anti-glycan antibody assay, serum samples were tested by the GlycoChip^{®} and by a commercial ASCA kit (Inova Diagnostics, San Diego, CA). The ASCA results were compared with binding levels of antibodies against mannan residues on the GlycoChip^{®} glycan array. The two assays had a correlation of R=0.76, further validating the GlycoChip^{®} assay.

### Lack of correlation between Anti-Laminarobioside or Anti-Chitobiose Carbohydrate Antibodies (ALCA and ACCA) and anti mannan antibodies

The correlation between the CD patient cohort seroreactivities to mannan, laminarobioside and chitobioside were assessed, as different seroreactivities would suggest not only different epitopes but also the possibility of different patient subpopulations. There was a weak correlation between the levels of antibodies against mannan (IgG) and laminarobioside (IgG) (R²=0.26), mannan (IgG) and chitobioside (IgA) (R²=0.18), and laminarobioside (IgG) and chitobioside (IgA) (R²=0.03). This suggests that a minimal overlap exists between CD patients with antibodies against the glucose-based glycan laminarobioside and the chitin based glycan chitobiose, and anti-glycan antibodies may define different CD serotypes.

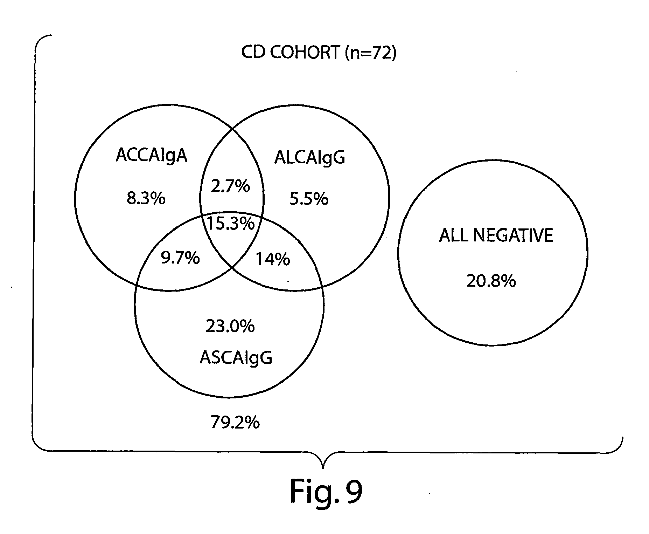

Twelve of 27 (44%) CD patients that were found to be ASCA negative were ALCA IgG or ACCA IgA positive (FIG. 9). The combined use of the three antigens may increase the sensitivity of differentiation between CD and UC up to 79%, while keeping specificity above 90%.

### OTHER EMBODIMENTS

It is to be understood that while the invention has been described in conjunction with the detailed description thereof, the foregoing description is intended to illustrate and not limit the scope of the invention, which is defined by the scope of the appended claims. Other aspects, advantages, and modifications are within the scope of the following claims.

**Table 1. Saccharides displayed on the glycan array**

| **Glycan** | **IUPAC** | **LINEARCODE®** | **Common Name** |
|---|---|---|---|
| 0 | p-Nitronhenol | pNP-0 | |
| 1 | Gal(α) | Aa | |
| 2 | Gal(β) | Ab | |
| 3 | Gal(β,1-3)GalNAc(α) | Ab3ANa | |
| 4 | Gal(β,1-3)GlcNAc(β) | Ab3GNb | |
| 5 | Gal(β,1-4)Glc(β) | Ab4Gb | Lactose |
| 6 | Gal(β,1-6)Gal(β) | Ab6Ab | |
| 7 | GalNAc(α) | ANa | |
| 8 | GalNAc(β) | ANb | |
| 9 | Fuc(α) | Fa | |
| 10 | Fuc(β) | Fb | |
| 11 | Glc(α) | Ga | |
| 12 | Glc(α,1-4)Glc(α) | Ga4Ga | Maltose |
| 13 | Glc(α,1-4)Glc(β) | Ga4Gb | |
| 14 | Glc(β) | Gb | |
| 15 | Glc(β,1-4)Glc(β) | Gb4Gb | Cellobiose |
| 16 | Glc(β,1-4)Glc(β,1-4)Glc(β) | Gb4Gb4Gb | Cellotriose |
| 17 | GlcNAc(α) | GNa | |
| 18 | GlcNAc(β) | GNb | |
| 19 | GlcNAc(β,1-3)GalNAc(α) | GNb3ANa | |
| 20 | GlcNAc(β,1-4)GlcNAc(β) | GNb4GNb | Chitobiose |
| 21 | L-Rha(α) | Ha | |
| 22 | GalA(β) | Lb | |
| 23 | Man(α) | Ma | |
| 24 | Man(β) | Mb | |
| 25 | Neu5Ac(α) | NNa | |
| 26 | L-Araf(α) | Ra | |
| 27 | GlcA(β) | Ub | |
| 28 | X(α) | Xa | |
| 29 | X(β) | Xb | |
| 30 | Gal(β,1-3)[GlcNAc(β,1-6)]GalNAc(α) | Ab3(GNb6)ANa | |
| 31 | Gal(β,1-4)GlcNAc(α) | Ab4GNa | |
| 32 | Gal(α,1-3)Gal(β,1-4)GlcNAc(β) | Aa3Ab4GNb | Linear B-2 |
| 33 | Gal(β,1-3)Gal(β,1-4)GalNAc(β) | Ab4GNb | |
| 34 | Man(β,1-4)GlcNAc(β) | Mb4Gb | |
| 35 | GlcNAc(β,1-6)GalNAc(α) | GNb6ANa | |
| 36 | Fuc(α,1-2)Gal(β) | Fa2Ab | |
| 37 | Man(α,1-3)Man(α) | Ma3Ma | |
| 38 | GlcNAc(β) 6-sulfate | GN[6S]b | |
| 39 | Glc(β,1-3)Glc(β) | Gb3Gb | |
| 40 | Gal(β) 3-sulfate | A[3S]b | |
| 41 | Man(α,1-3)[Man(α,1-6)]Man(β) | Ma3(Ma6)Mb | |
| 42 | GlcNAc(β,1-3)Gal(α,1-4)Glc(β) | GNb3Ab4Gb | Lacto-3 |
| 43 | Gal(α,1-4)Gal(β,1-4)Glc(β) | Aa4Ab4Gb | Pk antigen |
| 44 | Man(α,1-6)Man α | Ma6Ma | |
| 45 | Man(α,1-2)Man α | Ma2Ma | |
| 46 | Dextran | | Dextran |
| 47 | Mannam | | Mannam |
| 48 | Xylan | | Xylan |

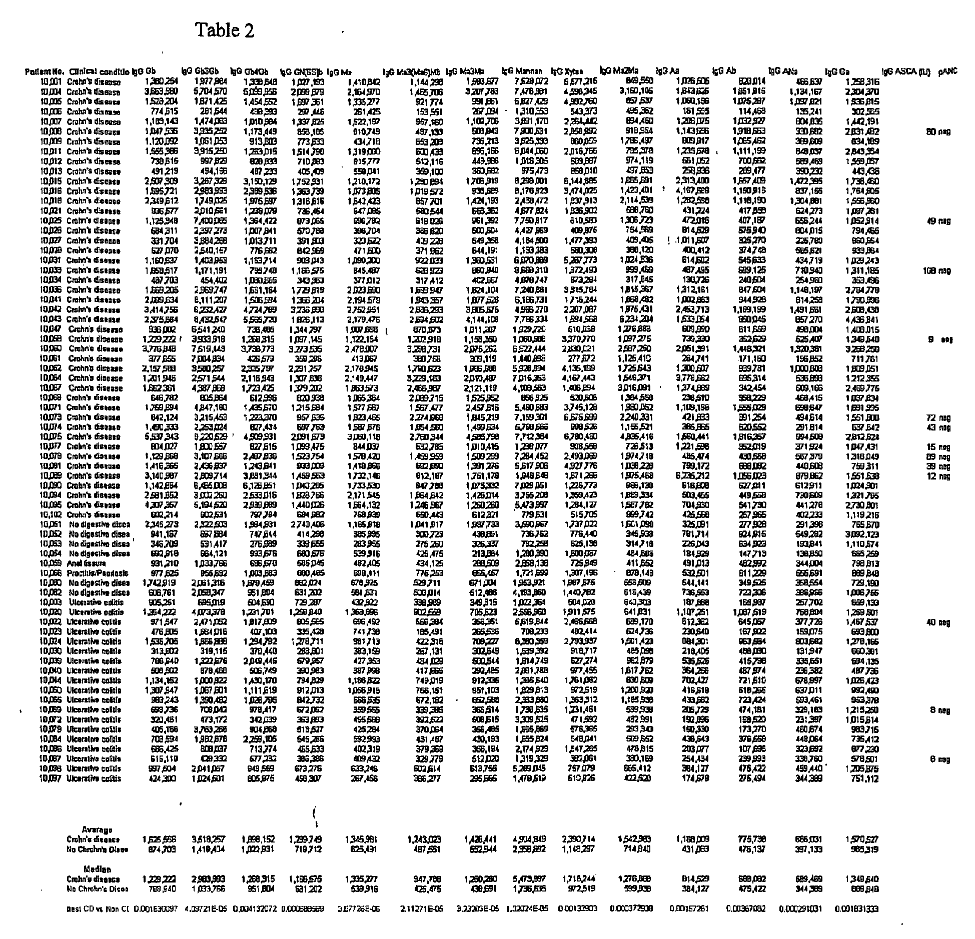

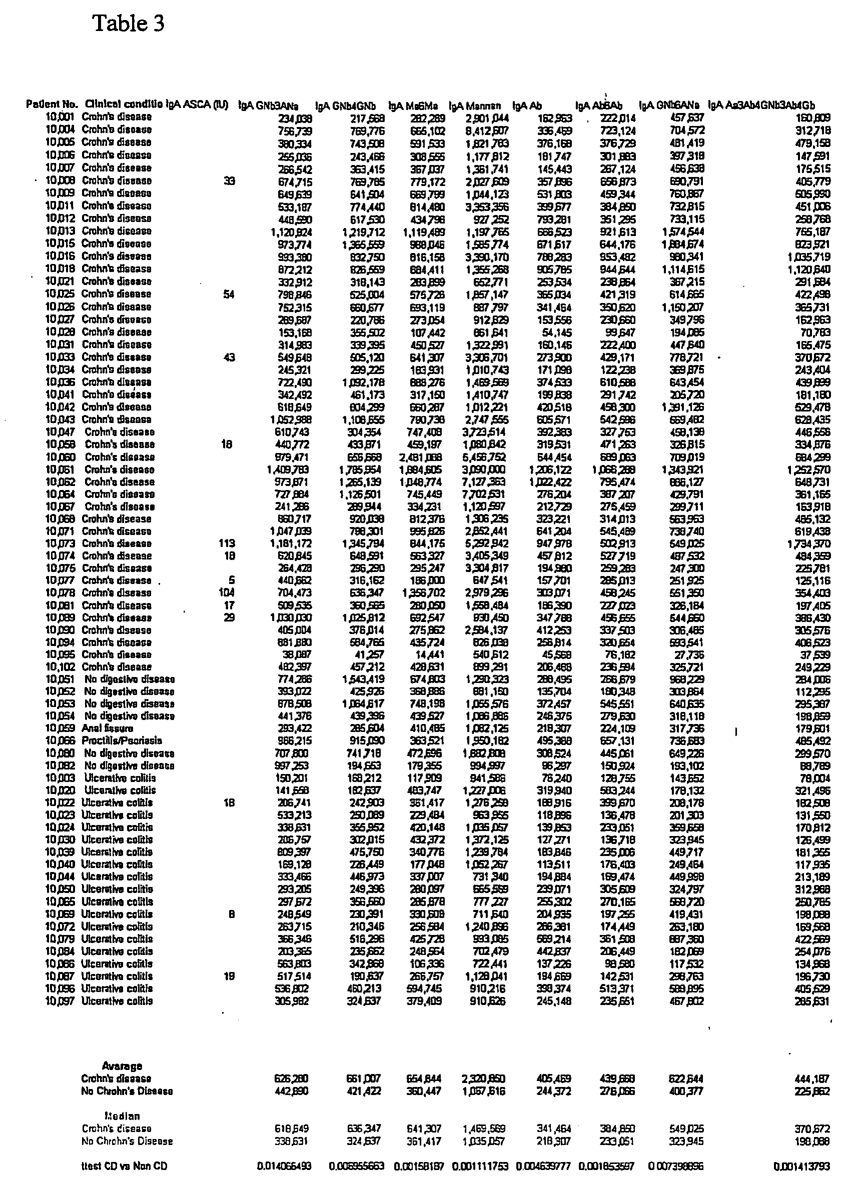

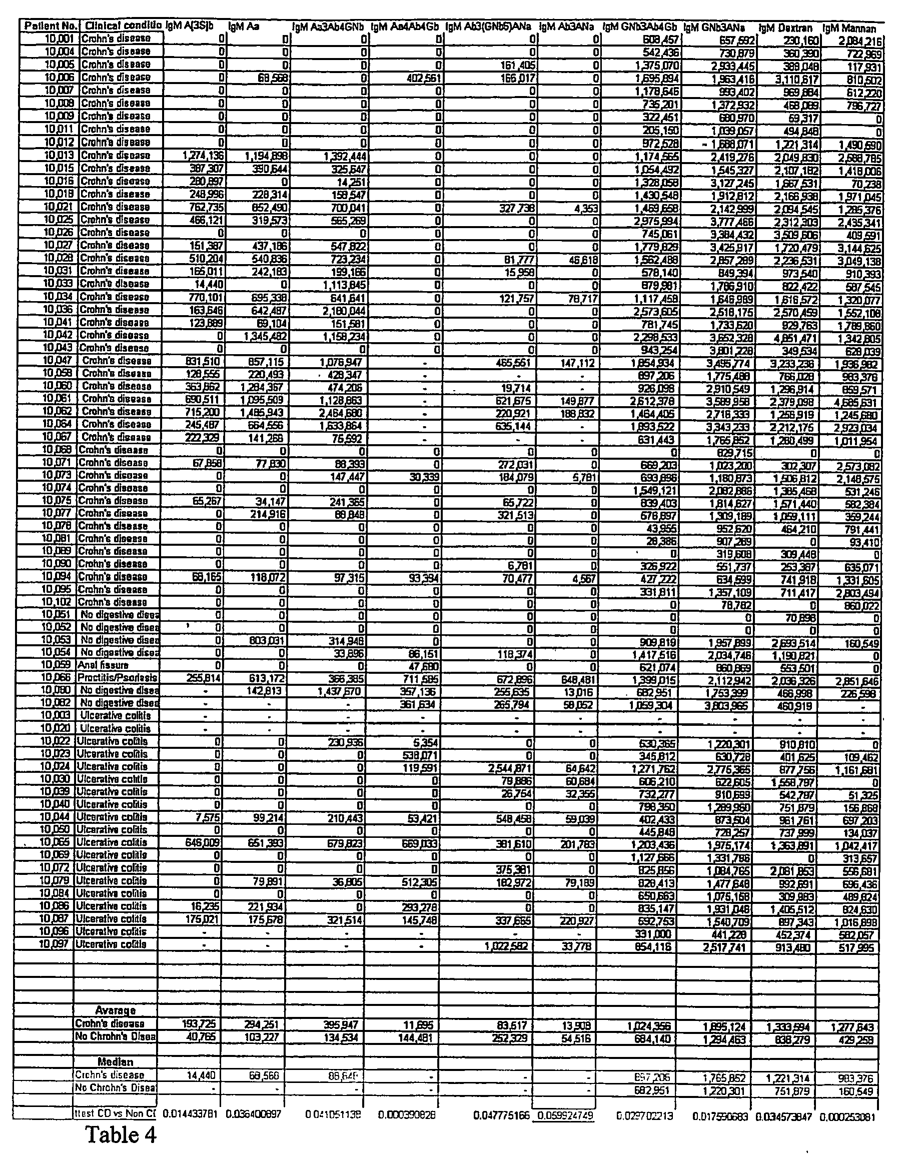

**Table 5 : Specificity and sensitivity of the different IgG anti glycans for differentiation between CD and other digestive diseases using different cut-off values. The cutoff values for each glycans where set as the 89 percentiles of the other digestive disease group. Glycans are presented in LINEARCODE®.**

| Cut-off level | | Anti Glycan IgG antibodies | | | | | | | | | |
|---|---|---|---|---|---|---|---|---|---|---|---|
| | | Gb | Gb3Gb | Gb4Gb | GGN[6S]b | Ma | Ma3(Ma6)Mb | MMa3Ma | Mannan | Xylan | Ma2Ma |
| | | | | | | | | | | | |
| 65^{th} percentile of non CD | Sensitivity for CD % | 76 | 73 | 62 | 62 | 58 | 60 | 71 | 78 | 58 | 71 |
| | Specificity for CD % | 70 | 63 | 67 | 70 | 67 | 78 | 70 | 67 | 63 | 67 |
| | | | | | | | | | | | |
| | | | | | | | | | | | |
| 75^{th} percentile of non CD | Sensitivity for CD % | 62 | 71 | 58 | 60 | 71 | 60 | 64 | 73 | 62 | 71 |
| | Specificity for CD % | 74 | 74 | 78 | 78 | 52 | 78 | 78 | 74 | 74 | 78 |
| | | | | | | | | | | | |
| | | | | | | | | | | | |
| 85^{th} percentile of non CD | Sensitivity for CD % | 56 | 64 | 51 | 49 | 49 | 56 | 62 | 67 | 40 | 62 |
| | Specificity for CD % | 81 | 81 | 81 | 81 | 85 | 85 | 81 | 81 | 81 | 85 |
| | | | | | | | | | | | |
| | | | | | | | | | | | |
| 90^{th} percentile of non CD | Sensitivity for CD % | 49 | 62 | 33 | 42 | 44 | 56 | 60 | 47 | 36 | 40 |
| | Specificity for CD % | 89 | 89 | 89 | 89 | 89 | 93 | 89 | 89 | 89 | 89 |

**Table 6: The sensitivity, specificity, True Positives (TP), True Negative (TN), False Positives (FP), and False Negatives (FN), and Positive Predictive Value (PPV) in different cut-of values for differentiation between CD and other digestive disease according to the level of Anti-Glc (β1-3) Glc (β) IgG.**

| IgG Gb3Gb | | | | | | | |
|---|---|---|---|---|---|---|---|
| (abnormate above cut-off) | Sensitivity | Specificity | TP | TN | FP | FN | PPV |
| - | 100.0% | 0.0% | 45 | 0 | 27 | 0 | 62.5 |
| 0 | 95.6% | 0.0% | 43 | 0 | 27 | 2 | 61.43 |
| 6,963 | 95.6% | 3.7% | 43 | 1 | 26 | 2 | 62.32 |
| 27,235 | 93.3% | 3.7% | 42 | 1 | 26 | 3 | 61.76 |
| 27,402 | 91.1% | 3.7% | 41 | 1 | 26 | 4 | 61.19 |
| 82,662 | 88.9% | 3.7% | 40 | 1 | 26 | 5 | 60.61 |
| 78,554 | 88.9% | 7.4% | 40 | 2 | 26 | 5 | 61.54 |
| 86,949 | 88.9% | 11.1% | 40 | 3 | 24 | 5 | 62.5 |
| 87,267 | 88.7% | 11.1% | 39 | 3 | 24 | 6 | 61.9 |
| 108,535 | 86.7% | 14.8% | 39 | 4 | 23 | 6 | 62.9 |
| 133,683 | 88.7% | 18.5% | 39 | 5 | 22 | 6 | 63.93 |
| 159,547 | 887% | 22.2% | 39 | 6 | 21 | 6 | 65 |
| 174,695 | 86.7% | 25.9% | 39 | 7 | 20 | 6 | 66.1 |
| 241,622 | 84.4% | 25.8% | 38 | 7 | 20 | 7 | 65.52 |
| 242,565 | 84.4% | 29.6% | 38 | 8 | 19 | 7 | 66.67 |
| 312,940 | 84.4% | 33.3% | 38 | 9 | 18 | 7 | 67.86 |
| 317,476 | 84.4% | 37.0% | 38 | 10 | 17 | 7 | 69.09 |
| 744,750 | 82.2% | 37.0% | 37 | 10 | 17 | 8 | 68.52 |
| 371,507 | 80.0% | 37.0% | 36 | 10 | 17 | 9 | 67.92 |
| 371,648 | 80.0% | 40.7% | 36 | 11 | 16 | 9 | 69.23 |
| 378,722 | 77.8% | 40.7% | 35 | 11 | 16 | 10 | 68.63 |
| 379,003 | 77.6% | 44.4% | 35 | 12 | 15 | 10 | 70 |
| 430,129 | 77.8% | 48.1% | 35 | 13 | 14 | 10 | 71.43 |
| 441,239 | 77.8% | 51.9% | 35 | 14 | 13 | 10 | 72.92 |
| 454,736 | 75.8% | 51.9% | 34 | 14 | 13 | 11 | 72.34 |
| 489,733 | 75.6% | 55.6% | 34 | 15 | 12 | 11 | 73.91 |
| 525,203 | 73.3% | 55.6% | 33 | 16 | 72 | 12 | 73.33 |
| 526,443 | 73.3% | 59.3% | 33 | 16 | 11 | 12 | 75 |
| 546,432 | 73.3% | 63.0% | 33 | 17 | 10 | 12 | 76.74 |
| 612,367 | 73.3% | 68.7% | 33 | 18 | 9 | 12 | 78.57 |
| 851,962 | 73.3% | 70.4% | 33 | 19 | 8 | 12 | 80.49 |
| 979,509 | 71.1% | 70.4% | 32 | 19 | 8 | 13 | 80 |
| 1,209,892 | 71.1% | 74.1% | 32 | 20 | 7 | 13 | 82.05 |
| 1,266,954 | 71.1% | 77.8% | 32 | 21 | 6 | 13 | 84.21 |
| 1,317,083 | 68.9% | 77.8% | 31 | 21 | 6 | 14 | 83.78 |
| 1,378,957 | 66.7% | 77.8% | 30 | 21 | 6 | 15 | 83.33 |
| 1,378,223 | 66.7% | 81.5% | 30 | 22 | 5 | 15 | 85.71 |
| 1,425,185 | 64.4% | 81.5% | 29 | 22 | 5 | 16 | 85.29 |
| 1,461,919 | 64.4% | 85.2% | 29 | 23 | 4 | 16 | 87.88 |
| 1,560,904 | 822% | 85.2% | 28 | 23 | 4 | 17 | 87.5 |
| 1,574,353 | 822% | 88.9% | 28 | 24 | 3 | 17 | 90.32 |
| 1,705,604 | 60.0% | 88.9% | 27 | 24 | 3 | 18 | 90 |
| 1,722,429 | 67.8% | 68.9% | 26 | 24 | 3 | 19 | 89.66 |
| 1,732,725 | 57.8% | 92.6% | 28 | 25 | 2 | 19 | 92.86 |
| 1,817,947 | 55.6% | 92.6% | 25 | 25 | 2 | 20 | 92.59 |
| 1,825,788 | 53.3% | 92.6% | 24 | 25 | 2 | 21 | 9231 |
| 1,869,774 | 51.1% | 92.6% | 23 | 25 | 2 | 22 | 92 |
| 1,880,984 | 48.9% | 92.6% | 22 | 25 | 2 | 23 | 91.67 |
| 1,994,899 | 46.7% | 92.6% | 21 | 25 | 2 | 24 | 91.3 |
| 2,067,775 | 44.4% | 92.6% | 20 | 25 | 2 | 25 | 90.91 |
| 2,154,175 | 422% | 92.6% | 19 | 25 | 2 | 26 | 90.48 |
| 2,324,221 | 40.0% | 92.6% | 18 | 25 | 2 | 27 | 90 |
| 2,467,429 | 37.8% | 92.6% | 17 | 25 | 2 | 28 | 89.47 |
| 2,551,776 | 35.6% | 92.6% | 16 | 25 | 2 | 29 | 88.89 |
| 2,703,085 | 35.6% | 96.3% | 16 | 28 | 1 | 29 | 94.12 |
| 2,850,072 | 33.3% | 98.3% | 15 | 26 | 1 | 30 | 93.75 |
| 3,096,078 | 31.1% | 96.3% | 14 | 26 | 1 | 31 | 93.33 |
| 3,186,273 | 28.9% | 96.3% | 13 | 26 | 1 | 32 | 92.86 |
| 3,441,678 | 28.9% | 100.0% | 13 | 27 | 0 | 32 | 100 |
| 3,511,076 | 26.7% | 100.0% | 12 | 27 | 0 | 33 | 100 |
| 3,559,430 | 24.4% | 100.0% | 71 | 27 | 0 | 34 | 100 |
| 3,578,889 | 222% | 100.0% | 10 | 27 | 0 | 35 | 100 |
| 4,137,076 | 20.0% | 100.0% | 9 | 27 | 0 | 36 | 100 |
| 4,327,530 | 17.8% | 100.0% | 8 | 27 | 0 | 37 | 100 |
| 5,107,549 | 15.6% | 100.0% | 7 | 27 | 0 | 38 | 100 |
| 5,545,432 | 13.3% | 100.0% | 6 | 27 | 0 | 39 | 100 |
| 5,640,050 | 11.1% | 100.0% | 5 | 27 | 0 | 40 | 100 |
| 5,724,798 | 8.9% | 100.0% | 4 | 27 | 0 | 41 | 100 |
| 6,708,583 | 6.7% | 100.0% | 3 | 27 | 0 | 42 | 100 |
| 6,891,638 | 4.4% | 100.0% | 2 | 27 | 0 | 43 | 100 |
| 7,209,245 | 22% | 100.0% | 1 | 27 | 0 | 44 | 100 |
| 7,299,442 | 0.0% | 100.0% | 0 | 27 | 0 | 45 | #### |

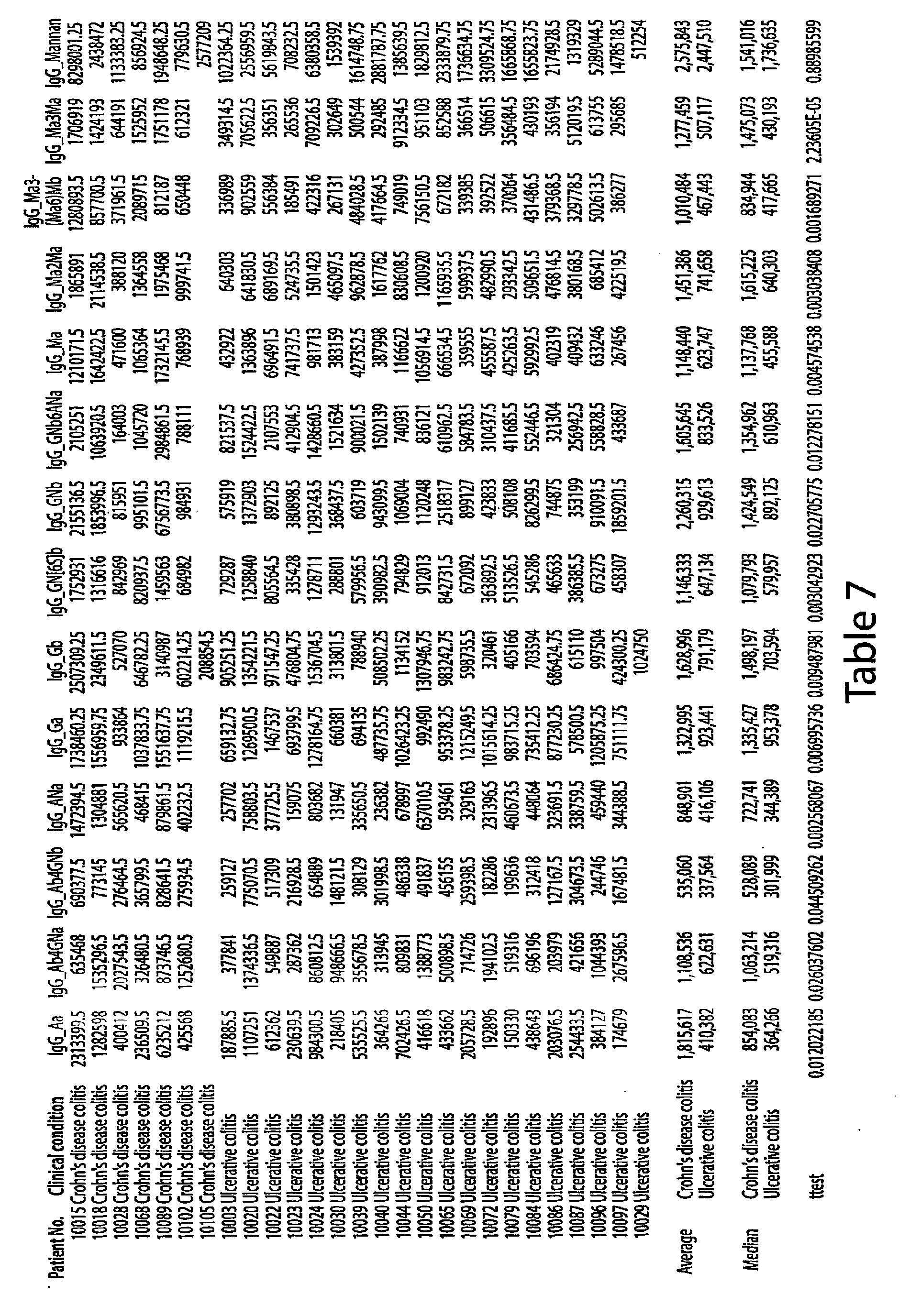

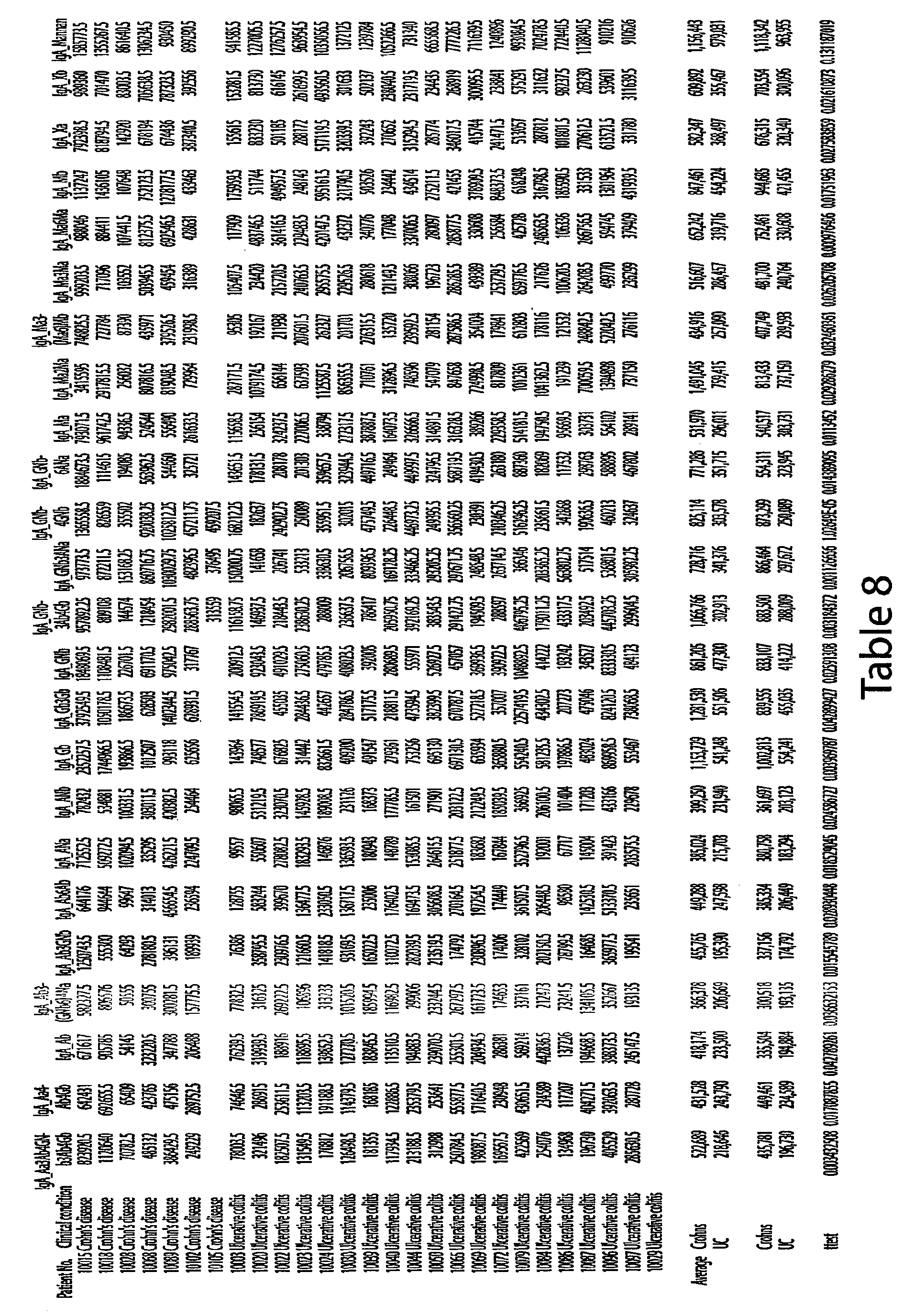

**Table 9**

| **Indication** | **Tests** | **Sensitivity, %** | **Specificity, %** |
|---|---|---|---|
| CD vs UC | Anti Mannan (ASCA) (+), or ALCA (+) | 71 | 91 |
| Non-IBD (NIC) vs IBD (UC+CD) | ACCA (+), and Anti Mannan (ASCA) (-) | 20 | 99 |

**Table 10. Patient characteristics**

| | CD (n=72) | UC (n=56) | Healthy control (n=41) |
|---|---|---|---|
| Age, year (range) | 35.5 (19-70) | 40.9 (14-89) | 38.9 (18-78) |
| Female, n (%) | 31 (43) | 28 (50) | 21 (51) |
| Mean disease duration, year (range) | 9.1 (1-54) | 7.9 (1-39) | -- |
| Mean age at diagnosis, year (range) | 26.6 (8-63) | 33 (10-87) | -- |
| Bowel surgery (including appendectomy), n (%) | 28 (38.9) | 3 (5.4) | -- |
| Extraintestinal manifestations, n (%) | 28 (38.9) | 14 (25.0) | -- |

| Treatment: | | | |
|---|---|---|---|
| 5-ASA, n (%) | 48(66.7) | 53(94.6) | -- |
| Antibiotics, n (%) | 15(20.8) | 4 (7.1) | -- |
| Steroids, n (%) | 23 (31.9) | 15(26.7) | -- |
| 6MP/AZA, n (%) | 22 (30.5) | 12 (21.4) | -- |
| MTX, n (%) | 5(6.9) | 0 (0) | -- |
| Infliximab, n (%) | 9 (12.5) | 0 (0) | -- |
| No treatment n (%) | 0 (0) | 0 (0) | -- |

| | | | |
|---|---|---|---|
| CD, Crohn's disease; UC, ulcerative colitis; HC, healthy control blood donors. | | | |

**Table 11**

| Glycan number | IUPAC nomenclature | Glycan number | IUPAC nomenclature |
|---|---|---|---|
| 1 | Gal(β) 3-sulfate | 26 | GlcNAc(β1-3)Gal(β1-4)Glc(β) |
| 2 | Gal(α) | 27 | GlcNAc(β1-6)GalNAc(α) |
| 3 | Gal(β) | 28 | L-Rha(α) |
| 4 | Gal(β1,3)[GlcNAc(β1,6)]GalNAc(α) | 29 | GalA(β) |
| 5 | Gal(β1-3)GalNAc(α) | 30 | Man(α) |
| 6 | Gal(β1-3)GlcNAc(β) | 31 | D-Manp(α1-3)D-Manp(α) |
| 7 | Gal(β-4)Glc(β) | 32 | Man(β) |
| 8 | Gal(β1-4)GlcNAc(α) | 33 | D-Manp(β1-4)D-Glcp(β) |
| 9 | Gal(β1-3)Gal(β1-4)GalNAc(β) | 34 | Neu5Ac(α) |
| 10 | Gal(β1-6)Gal(β) | 35 | L-Araf(α) |
| 11 | GalNAc(α) | 36 | GlcA(β) |
| 12 | GalNAc(β) | 37 | X(α) |
| 13 | Fuc(α) | 38 | X(β) |
| 14 | L-Fucp(α1-2)D-Galp(β) | 39 | PNP |
| 15 | Fuc(β) | 40 | Gal(α1-3)Gal(β1-4)GlcNAc(β) |
| 16 | Glc(α) | 41 | Gal(α1-3)Gal(β1-4)GlcNAc(β1-3)Gal(β1-4)Glc(β) |
| 17 | Glc(α1-4)Glc(α) | 42 | Gal(α1-4)Gal(β1-4)Glc(β) |
| 18 | Glc(α1-4)Glc(β) | 43 | Glc(β1-4)Glc(β) |
| 19 | Glc(β) | 44 | Man(α1-2)Manα |
| 20 | Glc(β1-3)Glc(β) | 46 | Man(α1-3)[Man(α1-6)]Man(β) |
| 21 | GlcNAc(β) 6-sulfate | 46 | Man(α1-6)Manα |
| 22 | GlcNAc(α) | 47 | Mannan |
| 23 | GlcNAc(β) | 48 | Xylan |
| 24 | GlcNAc(β1-3)GalNAc(α) | 49 | Dextran |
| 25 | GlcNAc(β1-4)GlcNAc(β) | | |

**Table 12: Anti-glycans IgG**

| | **Signal RFU*10⁶** **Mean (SD)** | | |
|---|---|---|---|
| **Sugar Antigen** | **CD** | **UC** | **Healthy control** |
| **Glc(**β**1,3)Glc(**β**)** | 2.26 (1.99) | 0.91 (1.13)** | 1.56 (1.39)* |
| α-**Man** | 1.35 (0.70) | 0.62 (0.30)** | 0.62 (0.29)* |
| **Man(**α**1,3)[Man(**α**1,6)]Man (**β**)** | 1.24 (0.84) | 0.46 (0.18)** | 0.53 (0.25) |
| **Man(**α**1,3) Man(**α**)** | 1.42(0.98) | 0.50 (0.22)** | 0.66 (0.57) |
| **Mannan** | 4.91 (2.34) | 1.91 (1.28)** | 1.97 (1.28)** |
| **GlcNAc(**β**) 6-sulfate** | 1.24 (0.66) | 0.65 (0.28)** | 0.89 (0.77) |

| | | | |
|---|---|---|---|
| RFU, relative fluorescence units; CD, Crohn's disease; UC, ulcerative colitis; SD, standard deviation. * <0.05 *versus* CD ** <0.001 *versus* CD | | | |

**Table 13: Anti-Glycan IgA**

| | **Signal RFU* 10⁶** | | |
|---|---|---|---|
| | CD | UC | Healthy control |
| **Sugar Antigen** | | | |
| **GlcNAc(**β**1,4)GlcNAc(**β**)** | 0.68 (0.48) | 0.39 (0.30)** | 0.50 (0.33) |
| **GlcNAc(**β**1,3)Gal(**β**1,4)Glc(**β**)** | 0.61 (0.51) | 0.39 (0.30) | 0.50 (0.33) |
| Mannan | 2.32 (1.89) | 0.97 (0.22)* | 1.27 (0.41) |

| | | | |
|---|---|---|---|
| RFU, relative fluorescence units; CD, Crohn's disease; UC, ulcerative colitis; SD, standard deviation. * <0.05 *versus* CD ** <0.001 *versus* CD | | | |

## Claims

1. A method useful in diagnosing Crohn's disease in a subject, the method comprising
identifying in a test sample from a subject with a symptom of Crohn's disease a specific anti-glycan antibody, wherein said specific anti-glycan antibody is an anti-GlcNAc(β,1-4)GlcNAc(β) antibody or an antl-Glc(β,1-3)Glc(β) antibody,
wherein the identification of elevated levels of said antibody in said test sample relative to a control sample indicates the subject is likely to have Crohn's disease.

2. The method of claim 1, wherein said method further comprises comparing levels of said specific anti-glycan antibody in said test sample to levels of said specific anti-glycan antibody in a control sample, wherein said control sample is selected from the group consisting of one or more individuals known to have or not to have a gastrointestinal disorder other than Crohn's disease.

3. The method of claim 2, wherein said control sample is from one or more individuals with a gastrointestinal disorder that is irritable bowel syndrome or ulcerative colitis.

4. The method of claim 2, wherein said control sample is from one or more individuals that do not have a gastrointestinal disorder.

5. The method of claim 1, further comprising determining whether said test sample has an anti-Mannan (ASCA) antibody, wherein the subject is assessed as having Crohn's disease if said anti-ASCA antibody is present in said sample.

6. The method of claim 1, further comprising determining whether said test sample has anti-neutrophil cytoplasmic antibodies (ANCA), wherein the subject is assessed as not having Crohn's Disease if said anti-neutrophil cytoplasmic antibodies (ANCA) are present in said sample.

7. The method of claim 5, further comprising determining whether said test sample has an anti-neutrophil cytoplasmic antibodies (ANCA), wherein the subject is assessed as not having Crohn's Disease if said anti-neutrophil cytoplasmic antibodies (ANCA) are present in said sample.

8. The method of claim 1, wherein said method further comprises identifying one, two, or three of an anti-Man(α,1-3) Man(a) antibody, anti-Man(α,1-3)[Man(α,1-6)]Man(α) antibody, anti-Man(α,1-2)Man(α), anti-Man(α,1-6)Man(α), or an anti-Mannan (ASCA ) antibody in said sample.

9. The method of claim 1, wherein said test sample is a biological fluid.

10. The method of claim 9, wherein said biological fluid is whole blood, serum, plasma, urine, or saliva.

11. The method of claim 9, wherein said wherein said biological fluid is serum.

12. The method of claim 1, further comprising determining the isotype of said antibody.

13. The method of claim 12, wherein said antibody is an IgM isotype antibody.

14. The method of claim 12, wherein said antibody is an IgA isotype antibody. .

15. The methods of claim 12, wherein said antibody is an IgG isotype antibody.

16. The method of claim 15, wherein said IgG antibody is an anti-Glc(β) antibody, an anti-Glc(β,1-3)Glc(β) antibody, an anti-Glc(β,1-4)Glc(β) antibody, an anti-GlcNAc(β) 6-sulfate antibody, or an anti-Xylan antibody.

17. The method of claim 1, wherein said specific anti-glycan antibody is identified with a fluorescent antibody.

18. The method of claim 1, wherein said specific anti-glycan antibody is identified with an enzyme-linked immunoabsorbent assay (ELISA).

19. The method of claim 1, wherein said antibody is an anti-Glc(β,1-3)Glc(β) antibody.

20. The method of claim 19, wherein said method comprises detecting an IgG anti-Glc(β,1-3)Glc(β) antibody in said sample.

21. The method of claim 20, wherein said method further comprises identifying an IgG anti-Man(α,1-3)Man(α) antibody in said sample.

22. The method of claim 19, wherein said method further comprises identifying an IgG anti-Man(α,1-3)Man(α) antibody in said sample.

23. The method of claim 19, wherein said method further comprises determining whether said sample has an IgG anti-Mannan or an IgA anti-Mannan antibody, wherein said subject is assessed as having Crohn's disease if said IgG anti-Mannan or IgA anti-Mannan antibody is present in said sample.

24. The method of claim 23, wherein said method comprises determining whether said sample has an IgG anti-Mannan antibody.

25. The method of claim 23, wherein said method comprises determining whether said sample has an IgA anti-Mannan antibody.

26. The method of claim 23, wherein said method further comprises determining whether said sample has anti-neutrophil cytoplasmic antibodies (ANCA), wherein said subject is assessed as having Crohn's disease if said ANCA are absent in said sample.

## Patentansprüche

1. Verfahren, das zur Diagnose von Crohn-Krankheit bei einem Individuum brauchbar ist, wobei das Verfahren ein Identifizieren eines spezifischen anti-Glycan-Antikörpers in einer Testprobe von einem Individuum mit einem Symptom der Crohn-Krankheit aufweist, wobei der spezifische anti-Glycan-Antikörper ein anti-GlcNAc(β, 1-4)GlcNAc(β)-Antikörper oder ein anti-Glc(β, 1-3)Glc(β)-Antikörper ist,
wobei das Identifizieren von erhöhten Spiegeln des Antikörpers in der Testprobe relativ zu einer Kontrollprobe anzeigt, dass das Individuum wahrscheinlich Crohn-Krankheit hat.

2. Verfahren nach Anspruch 1, bei dem das Verfahren außerdem ein Vergleichen von Spiegeln des spezifischen anti-Glycan-Antikörpers in der Testprobe mit Spiegeln des spezifischen anti-Glycan-Antikörpers in einer Kontrollprobe aufweist, wobei die Kontrollprobe ausgewählt wird aus der Gruppe, die aus einem oder mehreren Individuen besteht, von denen bekannt ist, dass sie eine andere gastrointestinale Erkrankung als Crohn-Krankheit haben oder nicht haben.

3. Verfahren nach Anspruch 2, bei dem die Kontrollprobe von einem oder mehreren Individuen mit einer gastrointestinalen Erkrankung, die Reizcolon oder Colitis ulcerosa ist, stammt.

4. Verfahren nach Anspruch 2, bei dem die Kontrollprobe von einem oder mehreren Individuen, die keine gastrointestinale Erkrankung haben, stammt.

5. Verfahren nach Anspruch 1, außerdem aufweisend ein Bestimmen, ob die Testprobe einen anti-Mannan (ASCA)-Antikörper hat, wobei das Individuum als Crohn-Krankheit habend beurteilt wird, wenn der anti-ASCA-Antikörper in der Probe vorliegt.

6. Verfahren nach Anspruch 1, außerdem aufweisend ein Bestimmen, ob die Testprobe anti-Neutrophilenzytoplasma-Antikörper (ANCA) hat, wobei das Individuum als Crohn-Krankheit nicht habend beurteilt wird, wenn die anti-Neutrophilenzytoplasma-Antikörper (ANCA) in der Probe vorliegen.

7. Verfahren nach Anspruch 5, außerdem aufweisend ein Bestimmen, ob die Testprobe anti-Neutrophilenzytoplasma-Antikörper (ANCA) hat, wobei das Individuum als Crohn-Krankheit nicht habend beurteilt wird, wenn die anti-Neutrophilenzytoplasma-Antikörper (ANCA) in der Probe vorliegen.

8. Verfahren nach Anspruch 1, bei dem das Verfahren außerdem ein Identifizieren von einem, von zwei oder von drei der Antikörper anti-Man(α, 1-3)Man(α)-Antikörper, anti-Man(α, 1-3)[Man(α, 1-6)]Man(α)-Antikörper, anti-Man(α, 1-2)Man(α), anti-Man(α, 1-6)Man(α), oder anti-Mannan (ASCA)-Antikörper in der Probe aufweist.

9. Verfahren nach Anspruch 1, bei dem die Testprobe ein biologisches Fluid ist.

10. Verfahren nach Anspruch 9, bei dem das biologische Fluid Vollblut, Serum, Plasma, Urin oder Speichel ist.

11. Verfahren nach Anspruch 9, bei dem das biologische Fluid Serum ist.

12. Verfahren nach Anspruch 1, außerdem aufweisend ein Bestimmen des Isotyps des Antikörpers.

13. Verfahren nach Anspruch 12, bei dem der Antikörper ein Antikörper vom IgM-Isotyp ist.

14. Verfahren nach Anspruch 12, bei dem der Antikörper ein Antikörper vom IgA-Isotyp ist.

15. Verfahren nach Anspruch 12, bei dem der Antikörper ein Antikörper vom IgG-Isotyp ist.

16. Verfahren nach Anspruch 15, bei dem der IgG-Antikörper ein anti-Glc(β)-Antikörper, ein anti-Glc(β, 1-3)Glc(β)-Antikörper, ein anti-Glc(β, 1-4)Glc(β)-Antikörper, ein anti-GlcNAc(β) 6-sulfat-Antikörper oder ein anti-Xylan-Antikörper ist.

17. Verfahren nach Anspruch 1, bei dem der spezifische anti-Glycan-Antikörper mit einem fluoreszierendem Antikörper identifiziert wird.

18. Verfahren nach Anspruch 1, bei dem spezifische anti-Glycan-Antikörper mit einem Festphasen-Enzymimmunoassay (ELISA) identifiziert wird.

19. Verfahren nach Anspruch 1, bei dem der Antikörper ein anti-Glc(β, 1-3)Glc(β)-Antikörper ist.

20. Verfahren nach Anspruch 19, bei dem das Verfahren ein Nachweisen eines IgG-anti-Glc(β, 1-3)Glc(β)-Antikörpers in der Probe aufweist.

21. Verfahren nach Anspruch 20, bei dem das Verfahren außerdem ein Identifizieren eines IgG-anti-Man(α, 1-3)Man(a)-Antikörpers in der Probe aufweist.

22. Verfahren nach Anspruch 19, bei dem das Verfahren außerdem ein Identifizieren eines IgG-anti-Man(α, 1-3)Man(α)-Antikörpers in der Probe aufweist.

23. Verfahren nach Anspruch 19, bei dem das Verfahren außerdem ein Bestimmen, ob die Probe einen IgG-anti-Mannan- oder einen IgA-anti-Mannan-Antikörper hat, aufweist, wobei das Individuum als Crohn-Krankheit habend beurteilt wird, wenn der IgG-anti-Mannan- oder IgA-anti-Mannan-Antikörper in der Probe vorliegt.

24. Verfahren nach Anspruch 23, bei dem das Verfahren ein Bestimmen aufweist, ob die Probe einen IgG-anti-Mannan-Antikörper hat.

25. Verfahren nach Anspruch 23, bei dem das Verfahren ein Bestimmen, ob die Probe einen IgA-anti-Mannan-Antikörper hat, aufweist.

26. Verfahren nach Anspruch 23, bei dem das Verfahren außerdem ein Bestimmen, ob die Probe anti-Neutrophilenzytoplasma-Antikörper (ANCA) hat, aufweist, wobei das Individuum als Crohn-Krankheit habend beurteilt wird, wenn die ANCA in der Probe fehlen.

## Revendications

1. Procédé utile pour diagnostiquer la maladie de Crohn chez un sujet, le procédé comprenant :
l'identification, dans un échantillon de test provenant d'un sujet présentant un symptôme de la maladie de Crohn, d'un anticorps anti-glycane spécifique, lequel anticorps anti-glycane spécifique est un anticorps anti-GlcNAc(β,1-4) GlcNAc(β) ou un anticorps anti-Glc(β,1-3)Glc(β),
dans lequel l'identification de niveaux élevés dudit anticorps dans ledit échantillon de test, par rapport à un échantillon témoin, indique que le sujet est susceptible d'avoir la maladie de Crohn.

2. Procédé selon la revendication 1, lequel procédé comprend en outre la comparaison des niveaux dudit anticorps anti-glycane spécifique dans ledit échantillon de test avec les niveaux dudit anticorps anti-glycane spécifique dans un échantillon témoin, dans lequel ledit échantillon témoin est choisi dans l'ensemble constitué d'un ou plusieurs individus connus pour avoir ou pour ne pas avoir un trouble gastro-intestinal autre que la maladie de Crohn.

3. Procédé selon la revendication 2, dans lequel ledit échantillon témoin provient d'un ou plusieurs individus ayant un trouble gastro-intestinal qui est le syndrome du côlon irritable ou la rectocolite ulcéro-hémorragique.

4. Procédé selon la revendication 2, dans lequel ledit échantillon témoin provient d'un ou plusieurs individus n'ayant pas de trouble gastro-intestinal.

5. Procédé selon la revendication 1, comprenant en outre l'opération consistant à déterminer si ledit échantillon de test a un anticorps anti-mannane (ASCA), dans lequel le sujet est évalué comme ayant la maladie de Crohn si ledit anticorps anti-ASCA est présent dans ledit échantillon.

6. Procédé selon la revendication 1, comprenant en outre l'opération consistant à déterminer si ledit échantillon de test a des anticorps anti-neutrophiles cytoplasmiques (ANCA), dans lequel le sujet est évalué comme n'ayant pas la maladie de Crohn si lesdits anticorps anti-neutrophiles cytoplasmiques (ANCA) sont présents dans ledit échantillon.

7. Procédé selon la revendication 5, comprenant en outre l'opération consistant à déterminer si ledit échantillon de test a des anticorps anti-neutrophiles cytoplasmiques (ANCA), dans lequel le sujet est évalué comme n'ayant pas la maladie de Crohn si lesdits anticorps anti-neutrophiles cytoplasmiques (ANCA) sont présents dans ledit échantillon.

8. Procédé selon la revendication 1, dans lequel ledit procédé comprend en outre l'identification d'un, de deux ou de trois parmi un anticorps anti-Man(α,1-3)Man(α), un anticorps anti-Man(α,1-3)[Man(α,1-6)]Man(α), anti-Man(α,1-2)Man(α), anti-Man(α,1-6)Man(α), et un anticorps anti-mannane (ASCA) dans ledit échantillon.

9. Procédé selon la revendication 1, dans lequel ledit échantillon de test est un fluide biologique.

10. Procédé selon la revendication 9, dans lequel ledit fluide biologique est du sang entier, du sérum, du plasma, de l'urine ou de la salive.

11. Procédé selon la revendication 9, dans lequel ledit fluide biologique est du sérum.

12. Procédé selon la revendication 1, comprenant en outre la détermination de l'isotype dudit anticorps.

13. Procédé selon la revendication 12, dans lequel ledit anticorps est un anticorps d'isotype IgM.

14. Procédé selon la revendication 12, dans lequel ledit anticorps est un anticorps d'isotype IgA.

15. Procédé selon la revendication 12, dans lequel ledit anticorps est un anticorps d'isotype IgG.

16. Procédé selon la revendication 15, dans lequel ledit anticorps IgG est un anticorps anti-Glc(β), un anticorps anti-Glc (β,1-3) Glc (β), un anticorps anti-Glc (β, 1-4) Glc (β) , un anticorps anti-GlcNAc (β) 6-sulfate, ou un anticorps anti-xylane.

17. Procédé selon la revendication 1, dans lequel ledit anticorps anti-glycane spécifique est identifié au moyen d'un anticorps fluorescent.

18. Procédé selon la revendication 1, dans lequel ledit anticorps anti-glycane spécifique est identifié au moyen d'un test par immunosorbant lié à un enzyme (ELISA).

19. Procédé selon la revendication 1, dans lequel ledit anticorps est un anticorps anti-Glc(β,1-3)Glc(β).

20. Procédé selon la revendication 19, lequel procédé comprend la détection d'un anticorps IgG anti-Glc(β,1-3)Glc(β) dans ledit échantillon.

21. Procédé selon la revendication 20, lequel procédé comprend en outre l'identification d'un anticorps IgG anti-Man(α,10-3)Man(α) dans ledit échantillon.

22. Procédé selon la revendication 19, lequel procédé comprend en outre l'identification d'un anticorps IgG anti-Man(α,1-3)Man(α) dans ledit échantillon.

23. Procédé selon la revendication 19, lequel procédé comprend en outre l'opération consistant à déterminer si ledit échantillon a un anticorps IgG anti-mannane ou IgA anti-mannane, dans lequel ledit sujet est évalué comme ayant la maladie de Crohn si ledit anticorps IgG anti-mannane ou IgA anti-mannane est présent dans ledit échantillon.

24. Procédé selon la revendication 23, lequel procédé comprend l'opération consistant à déterminer si ledit échantillon a un anticorps IgG anti-mannane.

25. Procédé selon la revendication 23, lequel procédé comprend l'opération consistant à déterminer si ledit échantillon a un anticorps IgA anti-mannane.

26. Procédé selon la revendication 23, lequel procédé comprend en outre l'opération consistant à déterminer si ledit échantillon a des anticorps anti-neutrophiles cytoplasmiques (ANCA), dans lequel ledit sujet est évalué comme ayant la maladie de Crohn si lesdits ANCA sont absents dans ledit échantillon.
